# EUROPÄISCHE PATENTSCHRIFT

(11) **EP 3 271 337 B1**
(45) Veröffentlichungstag und Bekanntmachung des Hinweises auf die Patenterteilung: **03.03.2021**
(21) Anmeldenummer: 16713747.0
(22) Anmeldetag: 15.03.2016
(51) Int. Cl.: C07D 239/22, C07C 317/32, A61K 31/513, A61P 9/00, A61P 9/12, A61P 11/00, A61P 17/02

(54) **VERFAHREN ZUR HERSTELLUNG VON (4S)-4-[4-CYANO-2-(METHYLSULFONYL)PHENYL]-3,6-DIMETHYL-2-OXO-1-[3-(TRIFLUORMETHYL)PHENYL]-1,2,3,4-TETRAHYDRO PYRIMIDIN-5-CARBONITRIL**
PROCESS FOR THE PREPARATION OF (4S)-4-[4-CYANO-2-(METHYLSULFONYL)PHENYL]-3,6-DIMETHYL-2-OXO-1-[3-(TRIFLUORMETHYL)PHENYL]-1,2,3,4-TETRAHYDRO PYRIMIDIN-5-CARBONITRIL
PROCÉDÉ DE PRÉPARATION DE (4S)-4-[4-CYANO-2-(METHYLSULFONYL)PHENYL]-3,6-DIMETHYL-2-OXO-1-[3-(TRIFLUORMETHYL)PHENYL]-1,2,3,4-TETRAHYDRO PYRIMIDIN-5-CARBONITRILE

(30) Priorität: 18.03.2015 EP 15159570
(43) Veröffentlichungstag der Anmeldung: 24.01.2018
(73) Patentinhaber: pH Pharma Co., Ltd., Jongno-Gu, Seoul 03142 (KR)
(72) Erfinder: SCHIRMER, Heiko, 42655 Solingen (DE); RUBENBAUER, Philipp, 40217 Düsseldorf (DE); KEIL, Birgit, 40231 Düsseldorf (DE); OLENIK, Britta, 46242 Bottrop (DE)
(74) Vertreter: Reitstötter Kinzebach
(86) Internationale Anmeldenummer: PCT/EP2016/055498
(87) Internationale Veröffentlichungsnummer: WO 2016/146607

(56) Entgegenhaltungen:
- WO-A1-2009/080199
- FRANZ VON NUSSBAUM ET AL: "Freezing the Bioactive Conformation to Boost Potency: The Identification of BAY 85-8501, a Selective and Potent Inhibitor of Human Neutrophil Elastase for Pulmonary Diseases", CHEMMEDCHEM, Bd. 10, Nr. 7, 17. Juni 2015 (2015-06-17), Seiten 1163-1173, XP055213187, ISSN: 1860-7179, DOI: 10.1002/cmdc.201500131 & Franz Von Nussbaum ET AL: "Supporting Information Freezing the Bioactive Conformation to Boost Potency: The Identification of BAY 85-8501, a Selective and Potent Inhibitor of Human Neutrophil Elastase for Pulmonary Diseases", , 17. Juni 2015 (2015-06-17), XP055270415, Gefunden im Internet: URL:http://onlinelibrary.wiley.com/store/1 0.1002/cmdc.201500131/asset/supinfo/cmdc_2 01500131_sm_miscellaneous_information.pdf? v=1&s=95066d173855b53e3fcd8578d6f689930a91 efd4 [gefunden am 2016-05-03]

## Beschreibung

Die vorliegende Anmeldung betrifft verbesserte Verfahren zur Herstellung von (4S)-4-[4-Cyano-2-(methylsulfonyl)phenyl]-3,6-dimethyl-2-oxo-1-[3-(trifluormethyl)phenyl]-1,2,3,4-tetrahydro pyrimidin-5-carbonitril und seiner Kristallform (A), die zur Herstellung von Arzneimitteln dient.

Die Verbindung (4S)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonitril ist aus WO 2009/080199 A1 bekannt und entspricht der Formel (I)

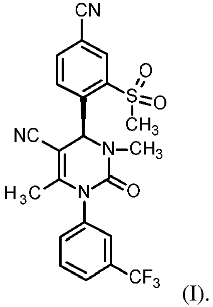

Bei der Verbindung der Formel (I) handelt es sich um einen Inhibitor der humanen Leukozyten-Elastase (HLE, EC 3.4.21.37), auch Humane Neutrophile Elastase (HNE, hNE) genannt. Die Humane Leukozyten-Elastase gehört zur Familie der Serinproteasen. Das proteolytische Enzym findet sich in den azurophilen Granula der polymorphkernigen Leukozyten (engl. *polymorphonuclear leukocytes, PMN leukocytes*). Die intrazelluläre Elastase nimmt eine wichtige Funktion in der Pathogenabwehr wahr, indem über Phagozytose aufgenommene Fremdpartikel abgebaut werden. Aktivierte neutrophile Zellen setzen die HNE aus den Granula in den Extrazellulärraum frei (extrazelluläre HNE), wobei ein Teil der freigesetzten HNE an der Außenseite der neutrophilen Zellmembran verbleibt (membranständige HNE). Das hochaktive Enzym ist in der Lage, eine Vielzahl von Bindegewebsproteinen abzubauen, z.B. die Proteine Elastin, Kollagen und Fibronektin. Elastin kommt in hohen Konzentrationen in allen Gewebetypen vor, die eine hohe Elastizität zeigen, z.B. in der Lunge und in Arterien. Bei einer Vielzahl von pathologischen Prozessen (z.B. Gewebeverletzungen) spielt die HNE eine Rolle beim Gewebeab- und -umbau (engl. *tissue remodeling*). Darüber hinaus ist die HNE ein wichtiger Modulator bei entzündlichen Prozessen. HNE induziert beispielsweise eine erhöhte Genexpression von Interleukin-8 (IL-8).

Es wird daher angenommen, dass die HNE bei vielen Erkrankungen, Verletzungen und pathologischen Veränderungen, deren Entstehung und/oder Progression mit einem entzündlichen Geschehen und/oder einem proliferativen und hypertrophen Gewebe- und Gefäßumbau in Zusammenhang steht, eine wichtige Rolle spielt. Dies können insbesondere Erkrankungen und/oder Schädigungen der Lunge oder des Herz-Kreislauf-Systems sein, oder es kann sich hierbei um eine Sepsis, um Krebs-Erkrankungen oder um andere entzündliche Erkrankungen handeln. HNE-Inhibitoren werden insbesondere zur Behandlung und/oder Prävention von Erkrankungen der Lunge und des Herz-Kreislauf-Systems verwendet.

In WO 2009/080199 A1 ist auch eine Methode zur Herstellung der Verbindung der Formel (I) beschrieben, die als nächster Stand der Technik angesehen wird. Hierbei wird ausgehend von 3-Fluor-4-methylbenzonitril die Zielverbindung (I) in 10 Stufen mit einer Gesamtausbeute von 4,45 % der Theorie hergestellt. Die Verbindung wird durch Eindampfen von Chromatographie-Fraktionen als amorpher Feststoff erhalten, ein definiertes Kristallisations-Verfahren der Endstufe zur Einstellung einer definierten Kristallform ist bisher nicht beschrieben.

Das nachfolgende Schema zeigt im Einzelnen die in der WO 2009/080199 A1 durchlaufenen Zwischenstufen.

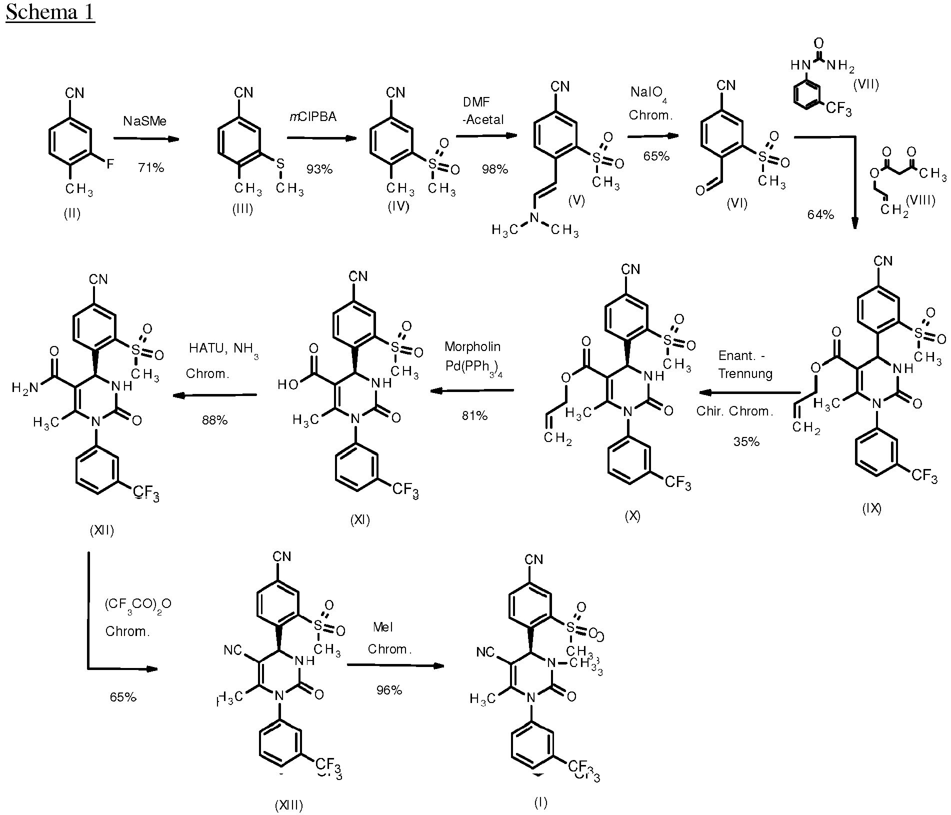

Das oben skizzierte Reaktionsschema ist in der WO 2009/080199 A1 wie folgt beschrieben: Die Reaktionssequenz von einer Verbindung der Formel (II) über die Verbindungen der Formeln (III), (IV) und (V) zu einer Verbindung der Formel (VI) in Schema 6 und den Beispielen 1A, 2A Methode B, 3A Methode B und 4A Methode B; die Reaktionssequenz von einer Verbindung der Formel (VI) über eine Verbindung der Formel (IX) zu einer Verbindung der Formel (X) in Schema 1 und den Beispielen 3 und 4; und die Reaktionssequenz von einer Verbindung der Formel (X) über die Verbindungen der Formeln (XI) und (XII) zu einer Verbindung der Formel (XIII) in Schema 2 und den Beispielen 5A, 5 und 6. Die Synthese der Verbindung der Formel (I) ist in Beispiel 33 Methode B beschrieben.

Es werden 4 chromatographische Aufreinigungen benutzt, sowie eine chirale Chromatographie-Stufe zur Auftrennung der Enantiomere (IX).

Dieses aus WO 2009/080199 A1 bekannte Verfahren weist verschiedene Nachteile in der Reaktionsführung auf, die sich besonders ungünstig bei der Herstellung der Verbindung der Formel (I) in technischem Maßstab auswirken.

Die Gesamtausbeute ist mit ca. 4,45 % der Theorie sehr gering. Viele Schritte verlaufen in sehr hoher Verdünnung und mit sehr hohen Reagenz-Überschüssen. So ist insbesondere die Sequenz zur Herstellung der Nitril-Aldehyd Zwischenstufe 4-Formyl-3-(methylsulfonyl)benzonitril (VI), die eine zentrale Rolle in dieser Synthese einnimmt, aus atomökonomischer Sicht nicht akzeptabel.

In der Synthese gemäß WO 2009/080199 A1 wurde der racemische Allylester der Formel (IX) mittels chiraler Chromatographie in die Enantiomeren aufgetrennt und dabei das S-Enantiomer (X) in 35% Ausbeute isoliert. Solch eine chromatographische Auftrennung der Racemate ist sehr kosten- und zeitintensiv und somit für eine Synthese in großtechnischem Maßstab unvorteilhaft.

Des Weiteren ist dieses in der WO 2009/080199 beschriebene Verfahren nicht in den technischen Maßstab zu übertragen, da zum einen sehr teure Reagenzien, wie Trifluoressigsäureanhydrid und *O*-(7-Azabenzotriazol-1-yl)-*N*,*N*,*N*,*N*'-tetramethyluronium-Hexafluorophosphat (HATU) eingesetzt werden. Trifluoressigsäureanhydrid wird für die Umsetzung der Verbindung der Formel (XII) zur Verbindung der Formel (XIII) verwendet, HATU wird für die Umsetzung der Verbindung der Formel (XI) zur Verbindung der Formel (XII) verwendet. Auch erlaubt eine Durchführung in technischem Maßstab keine toxischen Reagenzien. Dies ist *per se* von Nachteil, darüber hinaus müssen diese toxischen Substanzen aus dem Endprodukt (I) bis unterhalb der jeweils im Produkt aus regulatorischen Gründen zulässigen Höchstgrenze entfernt werden, was einen zusätzlichen Aufwand bedeutet. Dies betrifft vor allem die Alkylierung mit Methyliodid in fünffachem Überschuss als letzten Schritt der Synthesesequenz, da sichergestellt werden muss, dass das als karzinogen eingestufte Alkylierungsreagenz Methyliodid vollständig abgereinigt wird. Auch der Einsatz von Benzotriazolen, z.B. HATU, ist in großtechnischem Maßstab aus Gründen der Toxizität verboten. Des Weiteren werden gemäß dem in der WO 2009/080199 beschriebenen Verfahren viele chromatographische Zwischenreinigungen durchgeführt, die in der Regel sehr kostenintensiv sind. Es bestand daher der Bedarf an einer großtechnisch praktikablen Synthese, die die Verbindung der Formel (I) reproduzierbar in hoher Gesamtausbeute, niedrigen Gestehungskosten und hoher Reinheit liefert und allen regulatorischen Anforderungen entspricht, die einzuhalten sind, damit der Wirkstoff in klinischen Prüfungen eingesetzt und für eine spätere behördliche Einreichung verwendet werden kann. Vorteilhaft wäre auch, wenn man das nicht gewünschte Enantiomer isomerisieren, und das dabei gewonnene Racemat erneut dem Prozess zuführen könnte.

Überraschenderweise wurde nun ein sehr effizientes Verfahren zur Herstellung der Verbindung der Formel (I) gefunden, das die oben genannten Anforderungen erfüllt. Das neue erfindungsgemäße Verfahren (Verfahrensvariante (A) liefert die Zielverbindung (I) in 8 Stufen (siehe Schemata 7, 2 und 3, unten) in mehr als 17 % d. Th. Gesamtausbeute ohne eine chromatographische Aufreinigung von Zwischenstufen. Eine alternative Verfahrensvariante (B) (siehe Schemata 7, 4, 5 und 6, unten) des erfindungsgemäßen Verfahrens liefert die Zielverbindung (I) in 9 Stufen, ebenfalls ohne eine chromatographische Aufreinigung von Zwischenstufen, wobei die Gesamtausbeute wie unten beschrieben von der Reaktionsführung abhängt.

Gegenstand der vorliegenden Erfindung ist ein Verfahren zur Herstellung von Verbindungen der Formel (I) 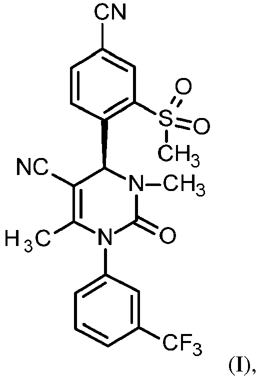 dadurch gekennzeichnet, dass man eine Verbindung der Formel (IX) 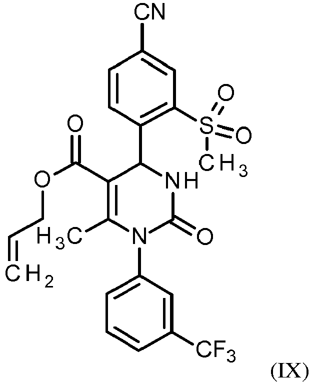
a-1) in Gegenwart eines Methylierungsmittels und einer Base zu einer Verbindung der Formel (XVI) 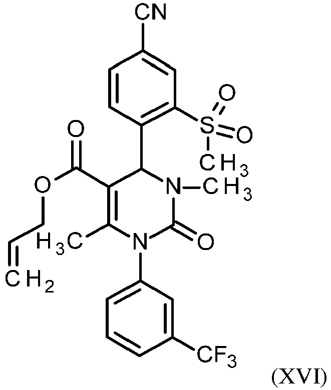 umsetzt; anschließend
a-2) eine Verbindung der Formel (XVI) in Gegenwart eines Palladiumkatalysators und einer sekundären Amin-Base zu einer Verbindung der Formel (XXVI) 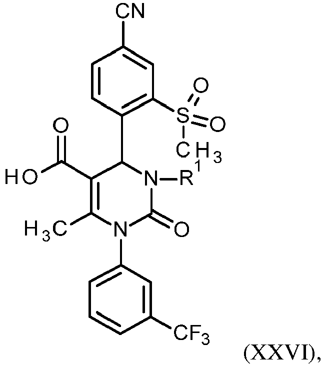 umsetzt, in welcher R¹ für Methyl steht,
   oder
b-1) in Gegenwart eines Palladiumkatalysators und einer sekundären Amin-Base zu einer Verbindung der Formel (XXVI) umsetzt, in welcher R¹ für Wasserstoff steht;
   anschließend
c) eine Verbindung der Formel (XXVI), in welcher R¹ für Wasserstoff oder Methyl steht, in Gegenwart eines China-Alkaloids und eines Lösungsmittels zu Verbindungen der Formeln (XXVIII) und (XXIX) 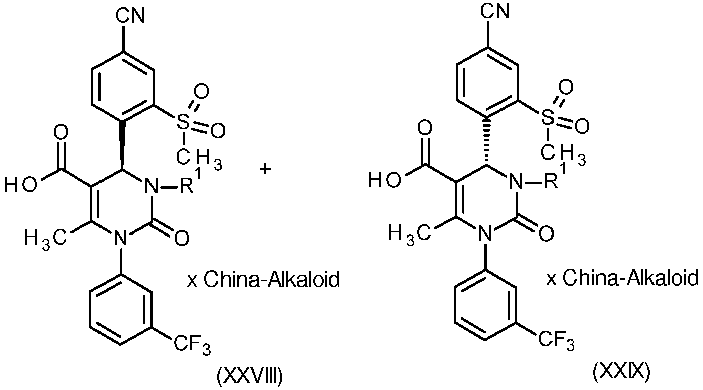 umsetzt, in welchen R¹ in Formel (XXVIII) und in Formel (XXIX) für Wasserstoff steht oder in welchen R¹ in Formel (XXVIII) und in Formel (XXIX) für Methyl steht; anschließend
d) eine Verbindung der Formel (XXVIII) isoliert; anschließend
e) eine Verbindung der Formel (XXVIII) in Gegenwart einer starken Säure zu einer Verbindung der Formel (XXVII) 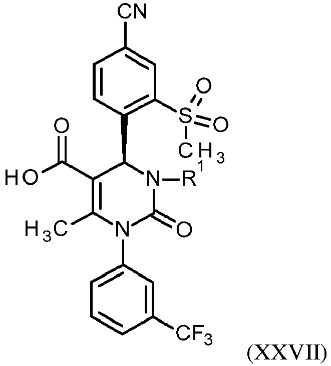 umsetzt, in welcher R¹ für Wasserstoff oder Methyl steht; anschließend
b-2) für den Fall dass R¹ in der Verbindung der Formel (XXVII) für Wasserstoff steht, eine Verbindung der Formel (XXVII) in Gegenwart eines Allyl-halogenids oder -sulfonats und einer Base zu einer Verbindung der Formel (X) 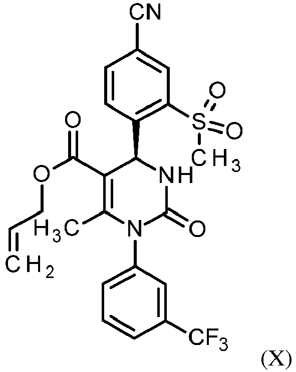 umsetzt, anschließend
b-3) eine Verbindung der Formel (X) in Gegenwart eines Methylierungsmittels und einer Base zu einer Verbindung der Formel (XXIII) 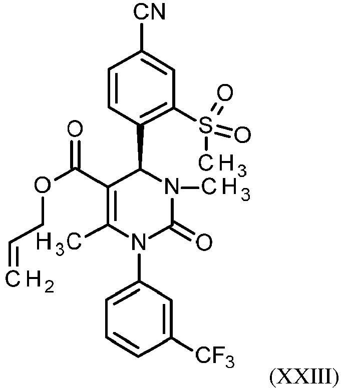 umsetzt; anschließend
b-4) eine Verbindung der Formel (XXIII) in Gegenwart eines Palladiumkatalysators und einer Base zu einer Verbindung der Formel (XXVII) 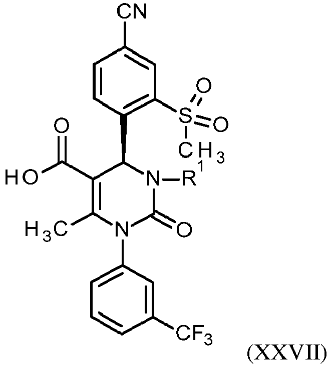 umsetzt, in welcher R¹ für Methyl steht; anschließend
f) eine Verbindung der Formel (XXVII), in welcher R¹ für Methyl steht, in Gegenwart eines Aktivierungsreagenzes zu einer Verbindung der Formel (XIX) 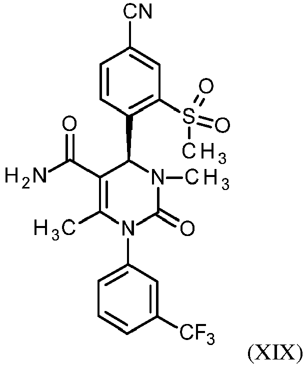 umsetzt; anschließend
g) eine Verbindung der Formel (XIX) in Gegenwart eines Dehydratisierungsmittels zu einer Verbindung der Formel (I) umsetzt;
   und gegebenenfalls im Anschluss an Reaktionsschritt c) eine Verbindung der Formel (XXIX) isoliert 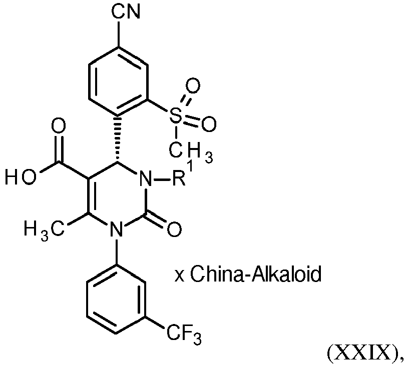 in der R¹ für Wasserstoff steht, diese gemäß Reaktionsschritt e) in Gegenwart einer starken Säure zu einer Verbindung der Formel (XXX) 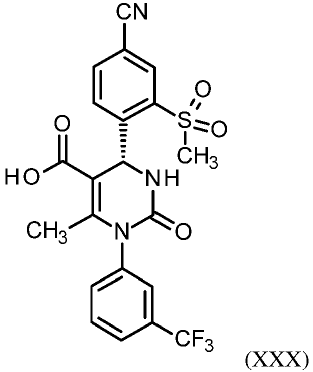 umsetzt; anschließend eine Verbindung der Formel (XXX) gemäß Reaktionsschritt b-2) in Gegenwart eines Allylhalogenids oder -sulfonats und einer Base zu einer Verbindung der Formel (XXXI) 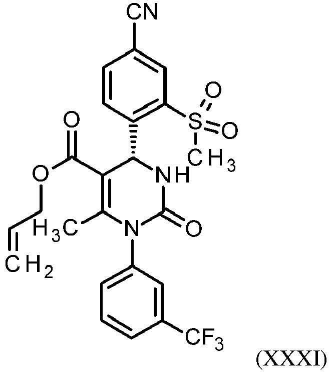 umsetzt; anschließend
h) eine Verbindung der Formel (XXXI) in Gegenwart einer starken, nicht nukleophilen Base in einem Lösungsmittel und unter gleichzeitigem Erwärmen in das Racemat der Formel (IX) 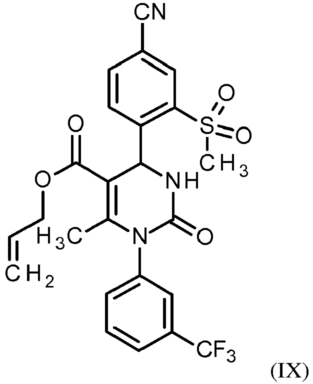 umsetzt; anschließend eine Verbindung der Formel (IX) gemäß den oben beschriebenen Reaktionsschritten b-1), c), d), e), b-2), b-3), b-4), f) und g) zu einer Verbindung der Formel (I) umsetzt;
   und gegebenenfalls die Reaktionsschritte der Isolierung einer Verbindung der Formel (XXIX) deren Umsetzung gemäß Reaktionsschritt e) in Gegenwart einer starken Säure zu einer Verbindung der Formel (XXX), die anschließende Umsetzung einer Verbindung der Formel (XXX) gemäß Reaktionsschritt b-2) in Gegenwart eines Allylhalogenids oder -sulfonats und einer Base zu einer Verbindung der Formel (XXXI), und anschließende Durchführung der Reaktionsschritte h), b-1), c), d), e), b-2), b-3), b-4), f) und g) einmal oder mehrmals wiederholt.

Das erfindungsgemäße Verfahren wird in zwei Verfahrensvarianten beschrieben. Verfahrensvariante (A) umfasst die oben beschriebenen Schritte a-1), a-2), c), d), e), f) und g). Die Racematspaltung, ein Schlüsselschritt der Synthese, findet in der Verfahrensvariante (A) auf der Stufe der Verbindung der Formel (XXVI) statt, in der R¹ für Methyl steht. Verfahrensvariante (B) umfasst die oben beschriebenen Schritte b-1), c), d), e), b-2), b-3), b-4), f) und g). Die Racematspaltung, ein Schlüsselschritt der Synthese, findet in der Verfahrensvariante (B) auf der Stufe der Verbindung der Formel (XXVI) statt, in der R¹ für Wasserstoff steht.

Gemäß einer Ausführungsform der vorliegenden Erfindung steht in den Formeln (XXVI), (XXVII), (XXVIII) und (XXIX) R¹ für Methyl und das Verfahren umfasst die oben beschriebenen Reaktionsschritte a-1), a-2), c), d), e), f) und g).

Gemäß einer Ausführungsform der vorliegenden Erfindung steht in den Formeln (XXVI), (XXVII), (XXVIII) und (XXIX) R¹ für Wasserstoff und das Verfahren umfasst die oben beschriebenen Reaktionsschritte b-1), c), d), e), b-2), b-3), b-4), f) und g).

Gemäß einer Ausführungsform der vorliegenden Erfindung steht in den Formeln (XXVI), (XXVII), (XXVIII) und (XXIX) R¹ für Wasserstoff und das Verfahren umfasst die oben beschriebenen Reaktionsschritte b-1), c), d), e), b-2), b-3), b-4), f) und g) und im Anschluss an Reaktionsschritt c) wird eine Verbindung der Formel (XXIX) isoliert, diese wird gemäß Reaktionsschritt e) in Gegenwart einer starken Säure zu einer Verbindung der Formel (XXX) umsetzt; anschließend wird eine Verbindung der Formel (XXX) gemäß Reaktionsschritt b-2) in Gegenwart eines Allylhalogenids oder -sulfonats und einer Base zu einer Verbindung der Formel (XXXI) umgesetzt; anschließend wird
h) eine Verbindung der Formel (XXXI) in Gegenwart einer starken, nicht nukleophilen Base in einem Lösungsmittel und unter gleichzeitigem Erwärmen in das Racemat der Formel (IX) umgesetzt; anschließend
wird eine Verbindung der Formel (IX) gemäß den oben beschriebenen Reaktionsschritten b-1), c), d), e), b-2), b-3), b-4), f) und g) zu einer Verbindung der Formel (I) umgesetzt;
und gegebenenfalls die Schritte der Isolierung einer Verbindung der Formel (XXIX) deren Umsetzung gemäß Reaktionsschritt e) in Gegenwart einer starken Säure zu einer Verbindung der Formel (XXX), die anschließende Umsetzung einer Verbindung der Formel (XXX) gemäß Reaktionsschritt b-2) in Gegenwart eines Allylhalogenids oder -sulfonats und einer Base zu einer Verbindung der Formel (XXXI), und anschließende Durchführung der oben beschriebenen Reaktionsschritte h), b-1), c), d), e), b-2), b-3), b-4), f) und g) einmal oder mehrmals wiederholt.

Methylierungsmittel, die für die Reaktionsschritte a-1) und b-3) verwendet werden können, sind beispielsweise Methyliodid, Dimethylsulfat, Dimethylcarbonat, Toluolsulfonsäuremethylester oder Methansulfonsäuremethylester, bevorzugt sind Methyliodid und Dimethylsulfat. Basen, die für die Reaktionsschritte a-1) und b-3) verwendet werden können sind beispielsweise Natriumhydrid, Natriumhexamethyldisilazan oder Lithiumhexamethyldisilazan, bevorzugt sind Natriumhexamethyldisilazan und Lithiumhexamethyldisilazan. Als Lösungsmittel wird für den Reaktionsschritt a-1) beispielsweise Tetrahydrofuran (THF) in 4-6 fachem Überschuss bezogen auf das Gewicht der Verbindung der Formel (IX) verwendet. Die Reaktionstemperatur beträgt -70 bis 40 °C, bevorzugt 0 bis 30 °C.

Gemäß einer Ausführungsform der vorliegenden Erfindung werden in den Reaktionsschritten a-1) und b-3) als Methylierungsmittel 2 eq. Dimethylsulfat (Me₂SO₄) und als Base Natrium-bis(trimethylsilyl)amid (NaHMDS) verwendet.

In der WO 2009/080199 A1 wird auch ein Syntheseweg beschrieben, welcher es erlaubt, die Methylgruppe schon auf der Allylesterstufe (X) einzuführen. Unter Verwendung von LiHMDS wurde bei einer Temperatur von -78 °C deprotoniert und anschließend durch Zugabe von 5 eq. Methyliodid die Methylgruppe eingeführt. Der methylierte S-Allylester wurde nach Aufarbeitung und chromatographischer Reinigung in einer Ausbeute von 59 % gewonnen (Beispiel 122). Die Verseifung zur entsprechenden Säure (XVIII) ist ebenfalls beschrieben (Beispiel 35A). Allerdings ist eine weitere Umsetzung zum Endprodukt der Formel (I) über das Amid (XIX) hier nicht beschrieben.

Mit dem Ziel, eine Alkylierung mit Methyliodid auf der Endstufe zu vermeiden, wird gemäß einer Ausführungsform des erfindungsgemäßen Verfahrens der racemische Allylester der Formel (IX) in Analogie zu der in der WO 2009/080199 A1 beschriebenen Synthese (Beispiel 122) methyliert. Im Gegensatz zu der in der WO 2009/080199 A1 beschriebenen Synthese erfolgt die Methylierung des Allylesters im erfindungsgemäßen Verfahren auf der Stufe des Racemats. Die erfindungsgemäße Reaktion weist gegenüber dem Stand der Technik signifikante Verbesserungen auf. Durch die Verwendung von NaHMDS als Base kann die Reaktion bei einer Temperatur von 20 °C in einem 4 bis 6 fachen Überschuss an THF, bezogen auf das Gewicht der Verbindung der Formel (IX), durchgeführt werden. Dies ist von Bedeutung für eine Synthese im großtechnischen Maßstab, da nun kein kostenintensiver Tieftemperaturreaktor benötigt wird. Außerdem kann das recht teure Alkylierungsreagenz Methyliodid durch das preiswerte Alkylierungsreagenz Dimethylsulfat ersetzt werden. Es werden 2 eq. Dimethylsulfat eingesetzt. Der Überschuss an Methylierungsmittel wird nach beendeter Reaktion durch Zugabe von wässriger Ammoniak-Lösung entfernt. Das Produkt (XVI) kann direkt aus der Reaktionsmischung durch Wasserfällung isoliert werden. Nach Isolierung wird im Vakuum getrocknet. Die Ausbeuten dieser Umsetzung sind in der Regel > 80 % d.Th..

Palladiumkatalysatoren, die für die Verseifung des Allylesters der Formel (XVI), (IX) oder (XXIII) gemäß Reaktionsschritten a-2), b-1) und b-4) verwendet werden können, sind beispielsweise Palladium-(0)-phosphankomplexe wie Tetrakis(triphenylphosphine)palladium(0) (Pd(PPh₃)₄) oder PalladiumAcetat/Triphenylphosphin (PdOAc₂/PPh₃), bevorzugt ist Pd-Acetat/Tripenylphosphin. Sekundäre Amin-Basen, die für den Reaktionsschritt a-2) verwendet werden können, sind beispielsweise Morpholin, Piperidin, Diisopropylamin oder N-Methyl-piperazin, bevorzugt sind Morpholin und N-Methyl-piperazin. Als Lösungsmittel wird für die Reaktionsschritte a-2), b-1) und b-4) beispielsweise Tetrahydrofuran (THF) in 3-5 fachem Überschuss, bezogen auf das Gewicht der Verbindung der Formel (XVI), (IX) bzw. (XXIII), verwendet.

Gemäß einer Ausführungsform der vorliegenden Erfindung wird in den Reaktionsschritten a-2), b-1) und b-4) als Palladiumkatalysator Palladiumacetat (PdOAc₂) mit Triphenylphosphin (PPh₃) als Liganden und als Base Morpholin verwendet.

Die Verseifung des racemischen Allylesters (XVI) zur freien Säure (XVII) erfolgt in Anlehnung an die in WO 2009/080199 A1 publizierte Vorschrift (Beispiel 5A, hier auf der Stufe des S-Enantiomers). Die erfindungsgemäße Reaktion weist gegenüber dem Stand der Technik signifikante Verbesserungen auf.

So wird der relativ teure sowie luftempfindliche Katalysator Palladiumtetrakistriphenylphosphin durch das stabile Palladiumacetat unter Zusatz von Triphenylphosphin als Ligand ersetzt. Weiterhin kann auch die Katalysatormenge von 0.05 eq. auf 0.003 eq. reduziert werden. Die palladiumkatalysierte Allylesterspaltung wird in einem 3 bis 5 fachen Überschuss an THF, bezogen auf das Gewicht der Verbindung der Formel (XVI), bei Temperaturen von 40 bis 60 °C in 1 bis 3 h unter Zugabe von Morpholin als Base durchgeführt. Das Produkt (XVII), welches als THF-Solvat anfällt, kann direkt aus der Reaktionsmischung durch Wasserfällung isoliert werden. Nach der Isolierung wird im Vakuum getrocknet. Die Ausbeuten dieser Umsetzung sind in der Regel > 95 % d.Th..

Die Racematspaltung der Säuren der Formel (XXVI), worin R¹ Wasserstoff oder Methyl ist, gemäß Reaktionsschritt c) der vorliegenden Erfindung ist ein Schlüsselschritt des erfindungsgemäßen Verfahrens zur Herstellung der Verbindung der Formel (I). China-Alkaloide, die für den Reaktionsschritt c) (Racematspaltung) verwendet werden können, sind ausgewählt aus der Gruppe bestehend aus Chinin, Chinidin, Cincholin und Cincholidin. Bevorzugt sind Chinin und Chinidin. Lösungsmittel, die für den Reaktionsschritt c) verwendet werden können, sind beispielsweise wässrige alkoholische Systeme, bevorzugt Isopropanol/Wasser, besonders bevorzugt Isopropanol/Wasser im Verhältnis 9:1. Als Lösungsmittel können für den Reaktionschritt c) außerdem Ester der Essigsäure, bevorzugt die C₂ bis C₅ Alkyl-Essigsäureester, besonders bevorzugt n-Butylacetat verwendet werden.

Gemäß einer Ausführungsform der vorliegenden Erfindung ist das China-Alkaloid für Reaktionsschritt c) ausgewählt aus der Gruppe bestehend aus Chinin und Chinidin.

Gemäß einer Ausführungsform der vorliegenden Erfindung ist das Lösungsmittel für Reaktionsschritt c) ausgewählt aus C₂-C₅-Alkylestern der Essigsäure, C₁-C₆ Alkoholen und Mischungen aus C₁-C₆ Alkoholen und Wasser.

Gemäß einer Ausführungsform der vorliegenden Erfindung wird für den Reaktionsschritt c) im Fall der Umsetzung von einer Verbindung der Formel (XXVI), in der R¹ für Methyl steht, eine Kombination aus Chinidin als China-Alkaloid und n-Butylacetat als Lösungsmittel verwendet. Die diastereomeren Chinidin-Salze der Verbindung der Formel (XXVI), in der R¹ für Methyl steht, können nach dem erfindungsgemäßen Verfahren in Lösungsmitteln wie den Estern der Essigsäure, bevorzugt die C₂ bis C₅ substituierten Ester, besonders bevorzugt n-Butylacetat, voneinander abgetrennt werden. So wird die racemische Verbindung der Formel (XXVI), in der R¹ für Methyl steht, beispielsweise in einem 4 bis 6 fachen Überschuss an Butylacetat, bezogen auf das Gewicht der Verbindung der Formel (XXVI mit R¹=Methyl), unter Zugabe von 1,0 bis 1,1 eq. Chinidin bei 40°C bis 60 °C umgesetzt. Dabei kristallisiert überwiegend das diastereomere Chinidinsalz der S-Säure (Verbindung der Formel (XX)) aus, während die R-Form (Verbindung der Formel (XXI)) in Lösung bleibt.

Zur Isolierung der Verbindung der Formel (XXVIII), in der R¹ für Wasserstoff oder Methyl steht, in Reaktionsschritt d) wird der Feststoff abfiltriert, mit dem in Reaktionsschritt c) verwendeten Lösungsmittel gewaschen und im Vakuum getrocknet.

Starke Säuren, die für den Reaktionsschritt e) verwendet werden können, sind beispielsweise wässrige Salzsäure, wässrige Bromwasserstoffsäure oder wässrige Schwefelsäure.

Gemäß einer Ausführungsform der vorliegenden Erfindung wird im Reaktionsschritt e) mit wässriger Salzsäure bis pH 1 angesäuert.

Gemäß einer Ausführungsform der vorliegenden Erfindung wird in Reaktionsschritt e) das Diastereomeren-reine Chinidinsalz (Verbindung der Formel (XX)) zur Freisetzung der Säure in Wasser suspendiert. Nach Ansäuern mit wässriger Salzsäure (bis pH = 1) verbleibt die Hilfsbase Chinidin als Hydrochlorid in Lösung, während die Enantiomeren-reine Säure (Verbindung der Formel (XXVII), in der R¹ für Methyl steht) ausfällt. Nach Isolierung wird im Vakuum getrocknet. Die Ausbeuten dieser Racematspaltung und Freisetzung zur S-Säure (Verbindung der Formel (XXVII), in der R¹ für Methyl steht) sind in der Regel > 40 % d.Th. mit einem Enantiomerenüberschuss von >98%.

Das andere Isomer kann ebenfalls aus der Mutterlauge der Racematspaltung durch Aufkonzentrieren und anschließende wässrige saure Aufarbeitung gewonnen werden.

Gemäß einer weiteren Ausführungsform der vorliegenden Erfindung wird für den Reaktionsschritt c) für den Fall der Umsetzung einer Verbindung der Formel (XXVI), in der R¹ für Wasserstoff steht, eine Kombination aus Chinin als China-Alkaloid und einer Mischung aus Isopropanol und Wasser als Lösungsmittel verwendet. Die diastereomeren Chinin-Salze der Säure der Formel (XXVI), in der R¹ für Wasserstoff steht, werden in wässrigen alkoholischen Systemen, bevorzugt Isopropanol/Wasser, besonders bevorzugt Isopropanol/Wasser im Verhältnis 9:1, voneinander abgetrennt. Gemäß dieser Ausführungsform wird die racemische Säure der Formel (XXVI), in der R¹ für Wasserstoff steht, in 6 bis 10 fachem Überschuss an Isopropanol/Wasser, bezogen auf das Gewicht der Verbindung der Formel (XXVI), unter Zugabe von 1,0 bis 1,1 eq. Chinin bei 40°C bis 60 °C umgesetzt. Dabei kristallisiert überwiegend das diastereomere Chininsalz der S-Säure (Verbindung der Formel (XXIV)), während die R-Form (Verbindung der Formel (XXV)) in Lösung bleibt.

Zur Isolierung der Verbindung der Formel (XXIV) gemäß Reaktionsschritt d) wird der Feststoff abfiltriert, mit Isopropanol/Wasser gewaschen und im Vakuum getrocknet.

Gemäß Schritt e) wird zur Freisetzung der Säure der Formel (XXVII), in der R¹ für Wasserstoff steht, das Diastereomeren-reine Chininsalz (XXIV) in Wasser suspendiert. Nach Ansäuern mit wässriger Salzsäure (bis pH = 1) verbleibt die Hilfsbase Chinin als Hydrochlorid in Lösung, während die Enantiomeren-reine Säure ausfällt. Nach Isolierung wird im Vakuum getrocknet. Die Ausbeuten dieser Racematspaltung und der Freisetzung zur S-Säure der Formel (XXVII), in der R¹ für Wasserstoff steht, sind in der Regel > 45 % d.Th. mit einem Enantiomerenüberschuss von >98%. Die Gesamtausbeute dieser Reaktionssequenz beträgt 18 %.

Das andere Isomer kann ebenfalls aus der Mutterlauge der Racematspaltung durch Aufkonzentrieren und anschließende wässrige saure Aufarbeitung gewonnen werden.

Der Erfolg einer solchen Racematspaltung ist in hohem Maß Substanz-abhängig und nicht vorhersehbar. Das in Reaktionsschritt c) beschriebene erfindungsgemäße Verfahren zur Racematspaltung ist daher überraschend.

Die Methylierung der Verbindung der Formel (XXVII), in der R¹ für Wasserstoff steht, kann nicht direkt mit der freien Carbonsäure der Formel (XXVII, R¹=Wasserstoff) durchgeführt werden. Daher muss die Carbonsäure der Formel (XXVII, R¹=Wasserstoff) erst in einen entsprechenden Ester, bevorzugt wieder in einen Allylester der Formel (X) überführt werden. Dies geschieht nach den dem Fachmann grundsätzlich bekannten Methoden gemäß Reaktionsschritt b-2) der vorliegenden Erfindung durch Alkylierung mit einem Allylhalogenid oder -sulfonat beispielsweise Allylbromid, Allylchlorid, Allyliodid, Allylmethansulfonat oder Allyltoluolsulfonat in Gegenwart von Basen beispielsweise Kaliumcarbonat, Natriumhydroxid, Kaliumhydroxid, Natriumcarbonat oder Natriumhydrid in Lösungsmitteln beispielsweise Aceton. So lässt sich die Carbonsäure (XI) in einer Ausbeute von 95% in den entsprechenden Allylester (X) überführen. Die folgenden Stufen zur Zielverbindung (I), werden analog zu dem in Variante (A) beschriebenen Verfahren durchgeführt.

Gemäß einer Ausführungsform wird für den Reaktionsschritt b-2) Allylbromid in Gegenwart von Kaliumcarbonat verwendet.

Die Bildung des Amides aus der Säure wurde in der Synthese gemäß WO 2009/080199 A1 unter Zuhilfenahme des recht teuren Amid-Kopplungsreagenzes *O*-(7-Azabenzotriazol-1-yl)-*N*,*N*,*N'*,*N'-*tetramethyluronium-Hexafluorophosphat (HATU) durchgeführt. Das Amid konnte erst nach chromatographischer Aufreinigung gewonnen werden. Es liegt auf der Hand, dass ein solches Verfahren nicht im großtechnischen Maßstab realisiert werden kann und somit ein hoher Bedarf an einer alternativen Verfahrensweise bestand.

Es wurde überraschenderweise gefunden, dass bei einer Umsetzung der Carbonsäure der Formel (XXVII, R¹=Methyl) in THF das Amid der Formel (XIX) direkt nach Wasserfällung aus der Reaktionslösung auskristallisiert und in hoher Ausbeute und Reinheit erhalten werden kann. Hierzu wird in Reaktionsschritt f) gemäß der vorliegenden Erfindung die Carbonsäure der Formel (XXVII, R¹=Methyl) mit einem Aktivierungsreagenz zunächst zum Imidazolid umgesetzt. Als Aktivierungsreagenz kann beispielsweise 1,1'-Carbonyldiimidazol zusammen mit Ammoniak oder 1,1'-Carbonyldiimidazol zusammen mit Hexamethyldisilazan eingesetzt werden.

Gemäß einer Ausführungsform der vorliegenden Erfindung wird die Carbonsäure der Formel (XXVII, R¹=Methyl) mit 1,2 bis 1,7 eq., bevorzugt 1,4 - 1,5 eq. 1,1'-Carbonyldiimidazol in THF bei Temperaturen zwischen 20 - 50 °C zunächst zum Imidazolid umgesetzt. Als bevorzugtes Vorgehen hat sich erwiesen, zuerst 1 bis 2 Stunden bei 20 °C zu rühren und dann 2 bis 3 Stunden bei 50 °C nachzurühren. Nach beendeter Aktivierung setzt man 5 - 20 eq., bevorzugt 10 eq. wässrige Ammoniaklösung zu und rührt 16-24 Stunden, bevorzugt 16 Stunden bei Raumtemperatur. Durch kurzes Erwärmen kann der überschüssige Ammoniak aus der Reaktionsmischung ausgegast werden. Zur Aufarbeitung wird die Reaktionslösung langsam auf Wasser dosiert. Dabei fällt das Produkt aus und kann durch Filtration oder Zentrifugieren isoliert werden. Man wäscht mit Wasser nach und trocknet im Vakuum bei erhöhter Temperatur (30 bis 100°C, bevorzugt bei 40°C bis 70°C). Die Ausbeuten sind sehr hoch und betragen in der Regel > 90% d. Theorie.

Die Bildung des Nitrils aus dem Amid wurde in der Synthese gemäß WO 2009/080199 A1 durch Entwässern mit 2 eq. Trifluoressigsäureanhydrid in THF durchgeführt. Das Nitril konnte erst nach chromatographischer Aufreinigung gewonnen werden. Es liegt auf der Hand, dass ein solches Verfahren nicht im großtechnischen Maßstab realisiert werden kann und somit ein hoher Bedarf nach einer alternativen Verfahrensweise bestand.

Geeignete Dehydratisierungsmittel zum Dehydratisieren von Amiden zu Nitrilen gemäß Reaktionsschritt g) des erfindungsgemäßen Verfahrens sind beispielsweise 1-Propanphosphonsäureanhydrid (T3P) oder Trifluoressigsäureanhydrid. Insbesondere hat sich 1-Propanphosphonsäureanhydrid (T3P) für diesen Reaktionsschritt bewährt. Dieses Reagenz kann als 50%ige Lösung in Ethylacetat bezogen werden. Es ist deutlich einfacher zu handhaben als das extrem hydrolyseempfindliche Trifluoressigsäureanhydrid. Hierzu wird das Amid der Formel (XIX) in Ethylacetat zunächst mit Diisopropylethylamin (Hünig-Base) und dann mit 1-Propanphosphonsäureanhydrid (T3P) versetzt. Zur Vervollständigung der Reaktion wird kurz unter Rückfluss erhitzt. Nach beendeter Reaktion wird mit Wasser versetzt und extrahiert. Anschließend wird die organische Phase mit gesättigter Natriumhydrogencarbonat-Lösung gewaschen und die organische Phase, die die Verbindung der Formel (I) enthält, abgetrennt.

Da die Verbindung der Formel (I) in Form einer Tablette entwickelt wird, besteht ein hoher Bedarf daran, dass man die isolierte Verbindung der Formel (I) reproduzierbar in einer definierten kristallinen Form isoliert, sodass man eine reproduzierbare Bioverfügbarkeit gewährleisten kann.

Eine Ausführungsform der Erfindung ist auch die Verbindung der Formel (I) in der Kristallform (A), dadurch gekennzeichnet, dass das Röntgendiffraktogramm der Verbindung Peakmaxima des 2 Theta Winkels bei 7.5, 12.4, 15.1, 18.5, 18.7, 22.9, 24.7 und 26.5 zeigt.

Gemäß einer Ausführungsform der Erfindung liefert das erfindungsgemäße Verfahren die Verbindung der Formel (I) in der Kristallform (A), dadurch gekennzeichnet, dass das Röntgendiffraktogramm der Verbindung der Formel (I) Peakmaxima des 2 Theta Winkels bei 7.5, 12.4, 15.1, 18.5, 18.7, 22.9, 24.7 und 26.5 zeigt.

Eine Ausführungsform der Erfindung ist auch die Verbindung der Formel (I) in der Kristallform (A), dadurch gekennzeichnet, dass das Raman Spektrum der Verbindung Bandenmaxima bei 3075, 2928, 2918, 2236, 2216, 1646, 1605, 1195 und 1004 cm⁻¹ zeigt.

Gemäß einer Ausführungsform der Erfindung liefert das erfindungsgemäße Verfahren die Verbindung der Formel (I) in der Kristallform (A), dadurch gekennzeichnet, dass das Raman Spektrum der Verbindung Bandenmaxima bei 3075, 2928, 2918, 2236, 2216, 1646, 1605, 1195 und 1004 cm⁻¹ zeigt.

Eine Ausführungsform der Erfindung ist ein Verfahren zur Herstellung der Verbindung der Formel (I) in der Kristallform (A), dadurch gekennzeichnet, dass eine Verbindung der Formel (I), vorliegend in einer oder mehreren Kristallformen oder als Solvat, in einem Alkohol, bevorzugt Ethanol, auskristallisiert wird, anschließend der dabei entstehenden Kristallbrei auf 50-80 °C erhitzt und 2-5 h bei dieser Temperatur nachgerührt wird.

Eine Ausführungsform der Erfindung ist die Verbindung der Formel (I) in der Kristallform (A) zur Behandlung von Krankheiten.

Eine Ausführungsform der Erfindung ist die Verbindung der Formel (I) in der Kristallform (A) zur Verwendung in einem Verfahren zur Behandlung und/oder Prävention von Erkrankungen der Lunge und des Herz-Kreislaufsystems und zur Förderung der Wundheilung, insbesondere bei chronischen Wunden.

Eine Ausführungsform der Erfindung ist die Verbindung der Formel (I) in der Kristallform (A) zur Verwendung in einem Verfahren zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD), der zystischen Fibrose (CF), der Bronchiektasie und zur Förderung der Wundheilung, insbesondere bei chronischen Wunden.

Eine Ausführungsform der Erfindung ist ein Arzneimittel enthaltend die Verbindung der Formel (I) in der Kristallform (A) in mehr als 90 Gewichtsprozente bezogen auf die Gesamtmenge der enthaltenen Verbindung der Formel (I).

Eine Ausführungsform der Erfindung ist die Verwendung der Verbindung der Formel (I) in der Kristallform (A) zur Herstellung eines Arzneimittels zur Behandlung von Erkrankungen der Lunge und des Herz-Kreislaufsystems und zur Förderung der Wundheilung, insbesondere bei chronischen Wunden.

Eine Ausführungsform der Erfindung ist ein Verfahren zur Behandlung von Erkrankungen der Lunge und des Herz-Kreislaufsystems und zur Förderung der Wundheilung, insbesondere bei chronischen Wunden durch Verabreichung einer wirksamen Menge der Verbindung der Formel (I) in der Kristallform (A).

Eine weitere Ausführungsform der Erfindung ist ein Verfahren zur Herstellung einer Verbindung der Formel (I) in der Kristallform (A), dadurch gekennzeichnet, dass man eine Verbindung der Formel (I) gemäß dem erfindungsgemäßen Verfahren herstellt, die Verbindung der Formel (I) aus der organische Phase aus Reaktionsschritt g) des erfindungsgemäßen Verfahrens in einem Alkohol, bevorzugt Ethanol, auskristallisiert, anschließend den dabei entstehenden Kristallbrei auf 50-80 °C erhitzt und 2-5 h bei dieser Temperatur nachrührt.

Für das abschließende Kristallisations-Verfahren wird aus GMP-technischen Gründen die ProduktLösung in Ethylester zuerst einer Partikelfiltration unterzogen und anschließend bei Rückflusstemperatur (60 °C - 80 °C) mit Ethanol versetzt, bevorzugt wird mit Toluol vergälltes Ethanol eingesetzt. Unter fortgesetzter Zugabe von Ethanol (Toluol-vergällt) wird der Ethylester abdestilliert. Dabei kristallisiert die Verbindung der Formel (I) aus. Um eine bessere Filtrierbarkeit zu gewährleisten, wird der Kristallbrei auf 60 °C - 80 °C erhitzt und 4 h bei dieser Temperatur nachgerührt. Es wird auf 20°C abgekühlt, anschließend werden die Kristalle isoliert und im Vakuum bei 40 - 50 °C getrocknet. Die Ausbeuten sind in der Regel > 80% d. Theorie. Die erzielte chemische Reinheit von > 99,2 % und der Gehalt von ca. 100 % entsprechen den Kriterien für Handelsprodukte nach ICH-Guideline. Die Menge an Restlösungsmittel, in diesem Fall Ethanol, ist < 0,1 %. Die optische Reinheit beträgt >> 99 % e.e.

Das Kristallisations-Verfahren ist sehr robust und liefert in reproduzierbarer Weise die gewünschte Kristallform (A) (Schmp. 232°C). Die Verbindung der Formel (I) wird in der Regel mikronisiert und in der Pharmazie zu Tabletten formuliert. Es zeigt sich, dass die Kristallform (A) sehr gute Stabilitäts-Eigenschaften besitzt, auch bei hoher Luftfeuchtigkeit, und über mehr als 3 Jahre hinweg ohne Stabilitätseinbußen gelagert werden kann.

Wie oben beschrieben kann der NH-Allylester der Formel (XXXI) durch Isolierung einer Verbindung der Formel (XXIX), deren Umsetzung gemäß Reaktionsschritt e) in Gegenwart einer starken Säure zu einer Verbindung der Formel (XXX) und anschließende Umsetzung einer Verbindung der Formel (XXX) gemäß Reaktionsschritt b-2) in Gegenwart eines Allylhalogenids oder -sulfonats und einer Base hergestellt werden. Dass dieser NH-Allylester der Formel (XXXI) racemisiert werden kann wurde überraschenderweise gefunden. Hiermit ist es möglich, das nicht gewünschte Enantiomer, welches in großen Mengen anfällt, wieder in die racemische Form umzuwandeln und somit wieder dem Prozess zuzuführen. Dazu wird die aus den Mutterlaugen der Racematspaltung gewonnene überwiegend R-haltige Carbonsäure zunächst unter den oben beschriebenen Bedingungen analog erfindungsgemäßem Schritt b-2) in einen Allylester überführt. Durch Behandeln mit starken, nicht nukleophilen Basen, beispielsweise 1,8-Diazabicyclo[5.4.0]undec-7-en (DBU) in Lösungsmitteln, beispielsweise THF, kann die überwiegend R-haltige Mischung durch Erwärmen über mehrere Stunden unter Rückfluss nahezu vollständig racemisiert werden. Nach Zugabe der Reaktionsmischung in Wasser fällt der racemische Allylester der Formel (IX) aus, wird isoliert und getrocknet. Der Allylester der Formel (IX) wird dann erneut unter den oben beschriebenen Bedingungen gemäß Reaktionsschritt b-1) zur Säure der Formel (XXVI, R¹ = Wasserstoff) verseift. Mit diesem Verfahren konnten 40% der zur Racematspaltung eingesetzten Menge an Säure der Formel (XXVI, R¹ = Wasserstoff) zurückgewonnen werden. Mit einem Rückgewinnungs-Zyklus konnte die Gesamtausbeute der Reaktionssequenz von ursprünglich 18 % auf 25 % gesteigert werden.

Eine weitere Ausführungsform der vorliegenden Beschreibung ist ein Verfahren zur Herstellung einer Verbindung der Formel (XXVII) 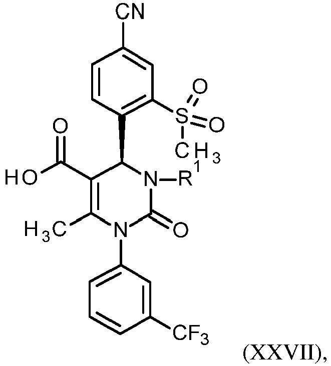 dadurch gekennzeichnet, dass man
c) eine Verbindung der Formel (XXVI) 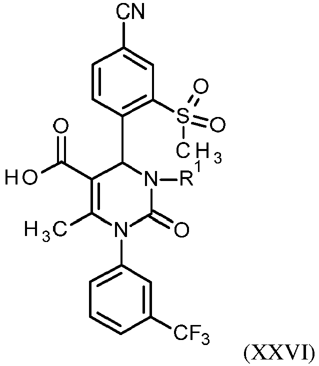 in Gegenwart eines China-Alkaloids und eines Lösungsmittels zu Verbindungen der Formeln (XXVIII) und (XXIX) 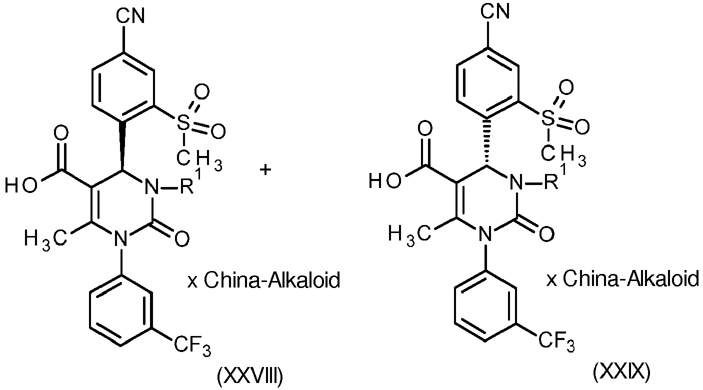 umsetzt; anschließend
d) eine Verbindung der Formel (XXVIII) isoliert; anschließend
e) eine Verbindung der Formel (XXVIII) in Gegenwart einer starken Säure in eine Verbindung der Formel (XXVII) umsetzt,
wobei R¹ in den Verbindungen der Formeln (XXVI), (XXVII), (XXVIII) und (XXIX) für Wasserstoff oder Methyl steht.

Die Reaktionsbedingungen für diese Reaktionssequenz sind wie oben beschrieben.

Eine weitere Ausführungsform der vorliegenden Beschreibung ist ein Verfahren zur Herstellung einer Verbindung der Formel (VI) 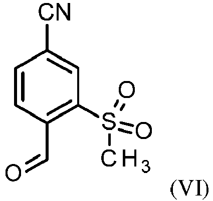 dadurch gekennzeichnet, dass man eine Verbindung der Formel (XV) 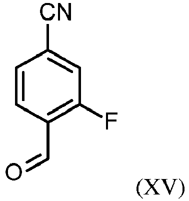 in Gegenwart von NaSO₂Me und DMSO oder Sulfolan bei 40-60 °C zu einer Verbindung der Formel (VI) umsetzt.

Gemäß einer Ausführungsform der vorliegenden Beschreibung wird die Umsetzung der Verbindung der Formel (XV) zur Verbindung der Formel (VI) in einem 3-5 fachen Überschuss an DMSO oder Sulfolan, bezogen auf das Gewicht der Verbindung der Formel (XV), durchgeführt.

Ein Vorteil dieser hohen Konzentration der Verbindung der Formel (XV) in dem Reaktionsansatz ist eine erhöhte Ökonomie des Verfahrens.

Als Ausgangsmaterial für 4-Formyl-3-(methylsulfonyl)benzonitril der Formel (VI) wird 4-Brom-2-Fluorbenzaldehyd der Formel (XIV) eingesetzt, welcher zunächst in an sich bekannter Weise nach dem Fachmann geläufigen Methoden (Synth. Commun. 1994, 887-890, Angew. Chemie 2003, 1700-1703, Tetrahedron Lett. 2007, 2555-2557, Tetrahedron Lett. 2004, 1441-1444, JACS 2003, 125, 2890-2891, Journal of Organometallic Chemistry 689 (2004), 4576-4583) in 3-Fluor-4-formylbenzonitril der Formel (XV) überführt wird. Besonders vorteilhaft hat sich erwiesen, eine Palladium katalysierte Umsetzung mit Kaliumhexacyanoferrat ^{∗} 3 H₂O als Cyanid-Quelle durchzuführen (Tetrahedron Lett. 48 (2007), 1087-1090). Hierzu wird 4-Brom-2-Fluorbenzaldehyd (XIV) in DMF (4- 6 facher Überschuss, bezogen auf das Gewicht der Verbindung der Formel (XIV)) vorgelegt, 0,22 eq. Kaliumhexacyanoferrat ^{∗} 3 H₂O und 1 eq. Natriumhydrogencarbonat vorgelegt und anschließend 0,005 eq. Palladiumacetat zugesetzt. Man erwärmt 3 Stunden auf 120°C. Die Lösung wird auf 20°C abgekühlt, anschließend Wasser und MtBE zugesetzt. Die organische Phase wird abgetrennt, die wässrige Phase wird erneut mit MtBE nachgewaschen und anschließend werden die vereinigten MtBE-Phasen unter Zugabe von Wasser aufkonzentriert. Dabei fällt das Produkt aus. Nach Isolierung wird im Vakuum getrocknet. Die Ausbeuten dieser Umsetzung sind in der Regel > 75 % d.Th.. 3-Fluor-4-formylbenzonitril (XV) ist mittlerweile auch kommerziell erhältlich.

Die Einführung einer Methylsulfonylgruppe in einen 2-Fluor substituierten Benzaldehyd ist beispielhaft in WO 2004/52858 (ELI LILLY) beschrieben. Die Umsetzung von 2-Fluorbenzaldehyd mit Natriummethansulfinat in DMSO bei 100 °C in 16 h lieferte das gewünschte Produkt jedoch nur in 50 % Ausbeute. Überraschenderweise wurde gefunden, dass sich 3-Fluor-4-formylbenzonitril (XV) schon unter relativ milden Reaktionsbedingungen (40-60°C, bevorzugt 50 °C, 4 h) in einem 3 bis 5-fachen Überschuss an DMSO, bezogen auf das Gewicht der Verbindung der Formel (XV), mit Natriummethansulfinat vollständig zum gewünschten 4-Formyl-3-(methylsulfonyl)benzonitril (VI) umsetzt. Das Produkt konnte direkt aus der Reaktionsmischung durch Wasserfällung isoliert werden. Nach Isolierung wird im Vakuum getrocknet. Die Ausbeuten dieser Umsetzung sind in der Regel > 90 % d.Th..

Somit wurde ein sehr effizienter Zugang zur Zwischenstufe 4-Formyl-3-(methylsulfonyl)benzonitril (VI) gefunden.

Das Kondensations-Produkt der Biginelli-Reaktion, (rac)-Allyl 4-(4-cyano-2--(methylsulfonyl)phenyl)-6-methyl-2-oxo-1-[3-(trifluormethyl)phenyl]-1,2,3,4-tetrahydropyrimidin-5-carboxylat (IX) aus 4-Formyl-3-(methylsulfonyl)benzonitril (VI), 1-[3-(Trifluormethyl)phenyl]harnstoff (VII) und Allyl-3-oxobutanoat (VIII) konnte in Anlehnung an die in WO 2009/080199 A1 publizierte Synthese-Vorschrift hergestellt werden. Auch hier konnte die Ausbeute signifikant von 64 % auf 87% gesteigert werden.

Gegenstand der vorliegenden Beschreibung sind auch Verbindungen der Formel (XXVII) 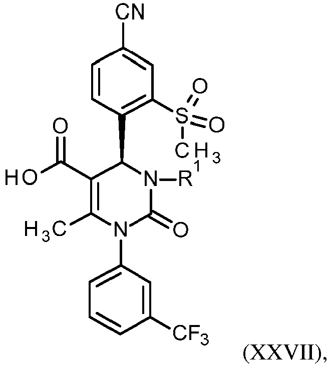 in welcher R¹ für Wasserstoff oder Methyl steht, sowie deren Salze und Solvate.

Das nachfolgende Schema 2 zeigt im Einzelnen die durchlaufenen Zwischenstufen von Verfahrensvariante (A).

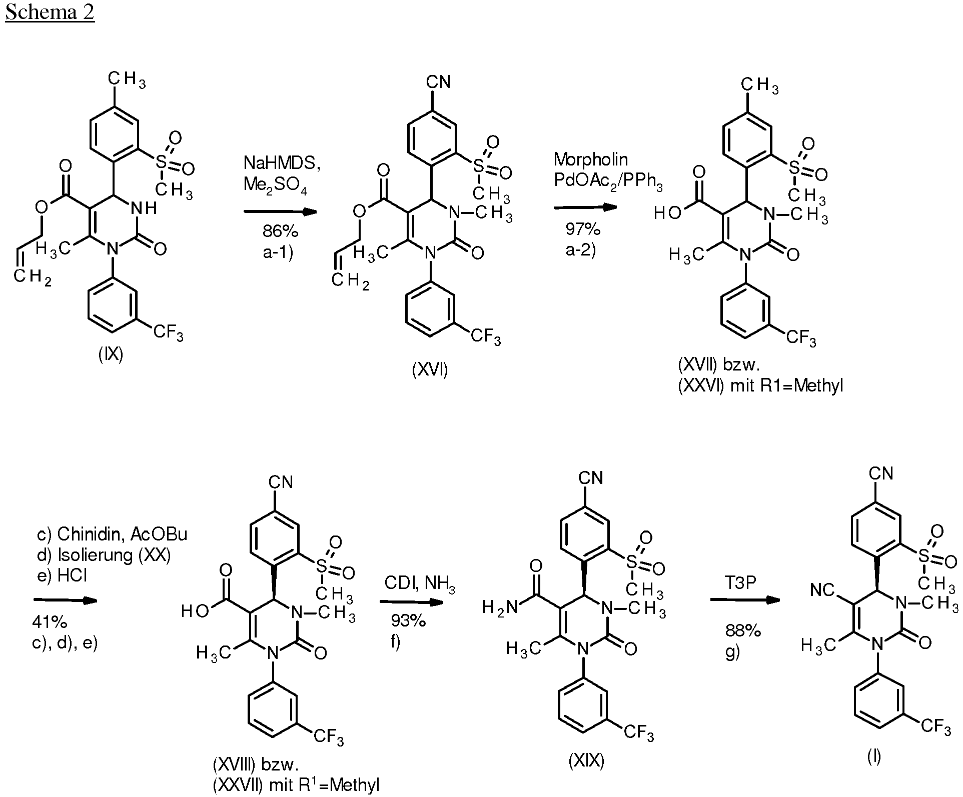

Gemäß einer Ausführungsform der vorliegenden Erfindung werden bei der Racematspaltung im Rahmen von Verfahrensvariante (A) die im nachfolgenden Schema 3 gezeigten Schritte durchlaufen.

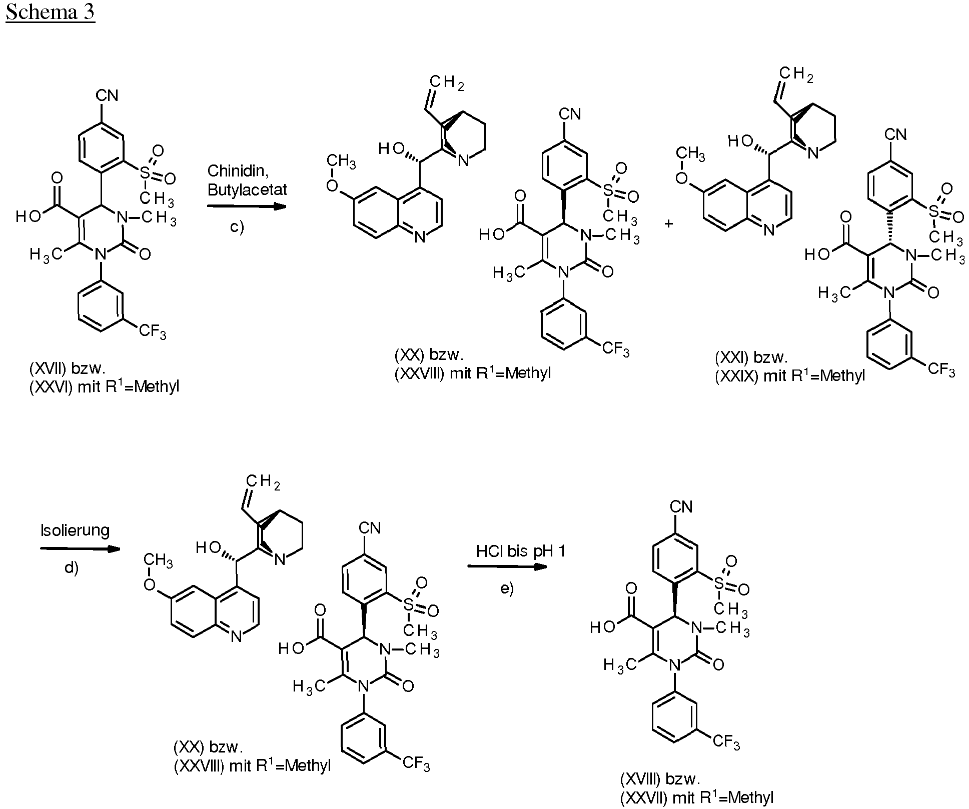

Das nachfolgende Schema 4 zeigt im Einzelnen die durchlaufenen Zwischenstufen von Verfahrensvariante (B):

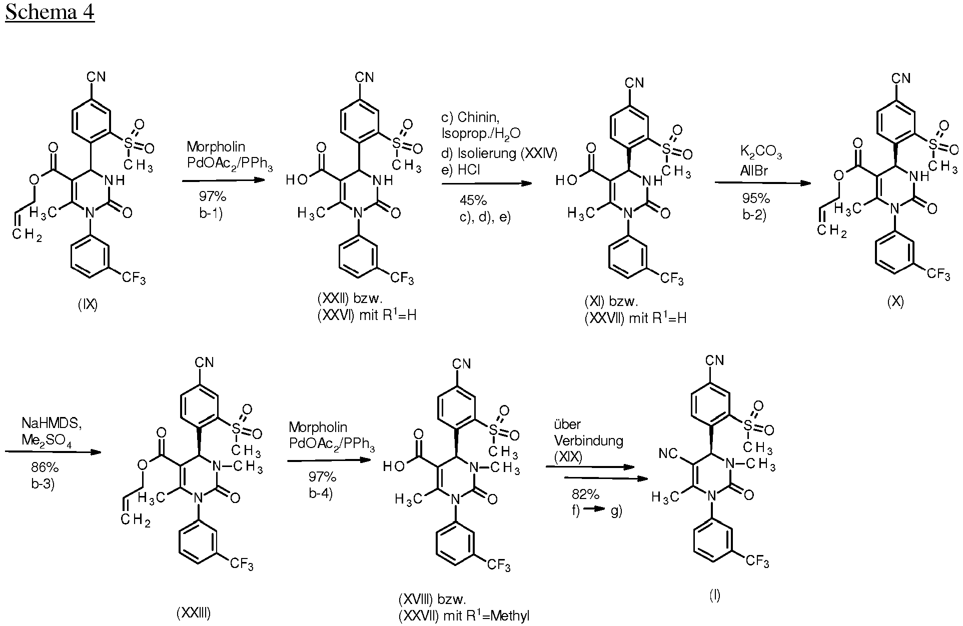

Gemäß einer Ausführungsform der vorliegenden Erfindung werden bei der Racematspaltung im Rahmen von Verfahrensvariante (B) die im nachfolgenden Schema 5 gezeigten Schritte durchlaufen.

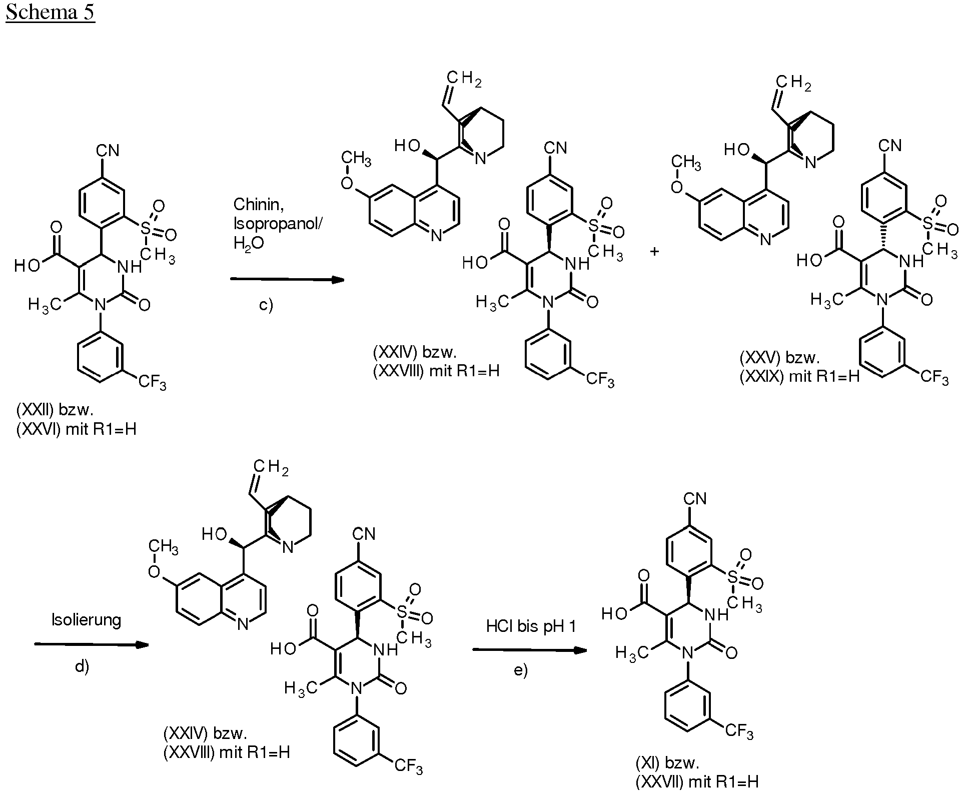

Gemäß einer Ausführungsform der vorliegenden Erfindung werden bei der Racematspaltung im Rahmen von Verfahrensvariante (B) die im nachfolgenden Schema 6 gezeigten Schritte durchlaufen, wobei das nicht gewünschte R-Isomer racemisiert und wieder dem Prozess zugeführt wird. Diese Reaktionssequenz kann gegebenenfalls einmal oder mehrmals wiederholt werden.

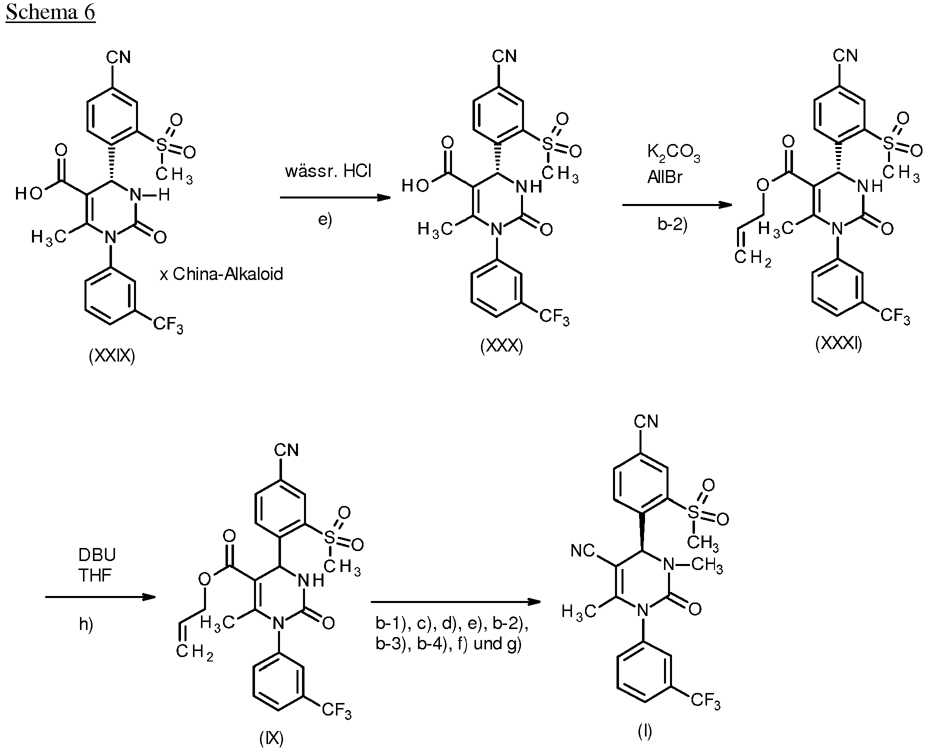

Die Reaktionsfolge der Synthese von 4-Brom-2-Fluorbenzaldehyd der Formel (XIV) bis zur Verbindung der Formel (IX) ist in folgendem Schema 7 dargestellt:

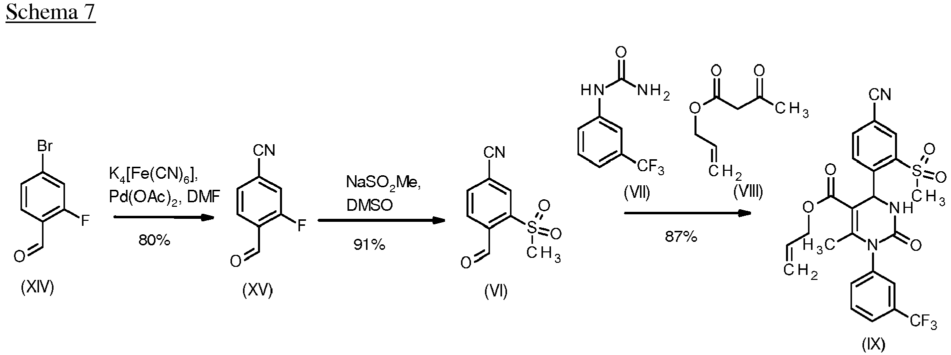

Mit der neuen erfinderischen Synthese ist es gelungen in sehr effizienter Weise die Zielverbindung (I) herzustellen. Das Verfahren bietet gegenüber dem Stand der Technik erhebliche Vorteile, was die Skalierbarkeit und technische Durchführung betrifft. Die Gesamtausbeute ist verglichen mit publizierten Daten signifikant höher, außerdem wird eine sehr hohe Reinheit des Wirkstoffs erzielt. Das neue Verfahren ermöglicht die reproduzierbare, ökonomische Herstellung der nicht im Stand der Technik vorbeschriebenen, definierten Kristallform (A). Mit dem hier vorgestellten erfinderischen Verfahren wurden bereits erfolgreich mehrere kg Material für klinische Prüfungen hergestellt.

### Abkürzungen:

- AllBr: Allylbromid
- aq.: wässrig, wässrige Lösung
- c: Konzentration
- cat.: katalytisch
- CDI: *N,N'*-Carbonyldiimidazol
- DBU: 1,8-Diazabicyclo[5.4.0]undec-7-en
- DCI: direkte chemische Ionisation (bei MS)
- dest.: destilliert
- DIEA: *N,N*-Diisopropylethylamin
- DMAP: 4-*N,N*-Dimethylaminopyridin
- DMF: Dimethylformamid
- DMSO: Dimethylsulfoxid
- d. Th.: der Theorie (bei Ausbeute)
- ee: Enantiomerenüberschuss
- ent: enantiomerenrein, Enantiomer
- eq.: Äquivalent(e)
- ESI: Elektrospray-Ionisation (bei MS)
- Et: Ethyl
- GC-MS: Gaschromatographie-gekoppelte Massenspektrometrie
- h: Stunde(n)
- HATU: *O*-(7-Azabenzotriazol-1-yl)-*N,N,N',N'*-tetramethyluronium-Hexafluorophosphat
- HPLC: Hochdruck-, Hochleistungsflüssigchromatographie
- konz.: konzentriert
- LC-MS: Flüssigchromatographie-gekoppelte Massenspektrometrie
- Me: Methyl
- min: Minute(n)
- MS: Massenspektrometrie
- MTBE: Methyl-*tert*.-butylether
- NaHMDS: Natrium-bis(trimethylsilyl)amid
- NMR: Kernresonanzspektrometrie
- Ph: Phenyl
- quant.: quantitativ (bei Ausbeute)
- rac: racemisch, Racemat
- RT: Raumtemperatur
- Rₜ: Retentionszeit (bei HPLC)
- Schmp.: Schmelzpunkt
- TFAA: Trifluoressigsäureanhydrid
- THF: Tetrahydrofuran
- T3P: 1-Propanphosphonsäureanhydrid
- UV: Ultraviolett-Spektrometrie
- v/v: Volumen zu Volumen-Verhältnis (einer Lösung)

### Ausführungsbeispiele:

### Beispiel 1

### 4-Formyl-3-fluorbenzonitril (XV)

400 g (1,97 mol) 4-Brom-2-fluorbenzaldehyd (XIV) als Lösung in 2,0 l DMF wurden mit 183 g (0,433 mol) Kaliumhexacyanoferrat (K₄[Fe(CN)₆]) und 165,5 g (1,97 mol) Natriumhydrogencarbonat vorgelegt und 2,2 g (9,85 mmol) Palladiumacetat zugegeben. Es wurde 2,5 Stunden bei 120°C gerührt. Es wurde auf 20°C abkühlen gelassen, dann wurden 2,0 l Wasser zum Ansatz gegeben. Es wurde mit 4,0 l MtBE extrahiert und die wässrige Phase wurde nochmals mit 1,5 l MtBE nachgewaschen. Die organischen Phasen wurden vereinigt und mit 2 l Wasser versetzt. Das MtBE wurde weitgehend bei 30 °C im leichten Vakuum abdestilliert. Dabei kristallisierte das Produkt aus. Es wurde auf 3°C abgekühlt und eine Stunde bei dieser Temperatur gerührt. Das Produkt wurde abfiltriert und mit Wasser (2 mal 0,8 l) nachgewaschen. Es wurde bei 40°C im Vakuum getrocknet. Ausbeute: 241 g (80 % d. Theorie) eines beigefarbenen Feststoffes.
MS (EIpos): m/z = 150 [M+H]+
1H-NMR (400 MHz, DMSO-d6): δ = 7.87 (d, 1H), 7.01 (s, 1H), 8,10 (d, 1H), 10.25 (s, 1H).

### Beispiel 2

### 4-Formyl-3-methylsulfonylbenzonitril (VI)

200 g (1,34 mol) 4-Formyl-2-fluorbenzonitril (XV) als Lösung in 0,8 l DMSO wurden vorgelegt und 192 g (1,88 mol) Methansulfinsäure Natriumsalz zugegeben. Es wurde 4 Stunden bei 50°C gerührt. Man ließ auf 20°C abkühlen. Das Reaktionsgemisch wurde auf 8,0 l Wasser zudosiert. Dabei kristallisierte das Produkt aus. Man rührte eine Stunde bei Raumtemperatur. Das Produkt wurde abfiltriert und mit Wasser (2 mal 0,1 l) nachgewaschen. Es wurde bei 40°C im Vakuum getrocknet. Ausbeute: 256 g (91 % d. Theorie) eines beigefarbenen Feststoffes.
MS (ESIpos): *m*/*z* (%) = 191.1 (15) [M-18]⁺, 161.0 (100).
1H-NMR (400 MHz, DMSO-d6): δ = 3,57 (s, 3H), 8,10 (d, 1H), 8,38 (d, 1H), 8,45 (s, 1H), 10.62 (s, 1H).

### Beispiel 3

### (rac)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (IX)

Zu Phosphorsäuretriethylester (124,3 g, 683 mmol) wurde Diphosphorpentoxid (64,6 g, 455 mmol) in 3 Portionen bei 20 °C zugegeben und 3 h bei 40°C gerührt. Dann wurde mit THF (115 ml) verdünnt, 30 min bei 20 °C nachgerührt, und es wurden 4-Formyl-3-(methylsulfonyl)benzonitril (VI) (119 g, 569 mmol) sowie 1-[3-(Trifluormethyl)phenyl]harnstoff (VII) (116 g, 569 mmol) zugegeben. Anschließend wurde während 20 min Allylacetoacetat (VIII) (121 g, 852 mmol) zudosiert, wobei die Temperatur auf ca. 60°C anstieg. Das Gemisch wurde 4 h bei 80 °C gerührt. Zur Aufarbeitung wurde bei 40 °C Wasser (115 ml) zugegeben und 30 min bei 25 °C gerührt. Das Produkt wurde abfiltriert und mit Wasser (280 ml) nachgewaschen. Der Rückstand wurde mit MtBE (280 ml) für 20 min verrührt, erneut abfiltriert und mit MtBE (220 ml) gewaschen. Es wurde bei 40°C im Vakuum getrocknet. Ausbeute: 259 g (87 % d. Theorie) eines beigefarbenen Feststoffes.
MS (ESIpos): *m*/*z* (%) = 520.2 (100) [M+H]⁺
¹H-NMR (400 MHz, DMSO-d₆): δ = 2.15 (s, 3H), 3.45 (s, 3H), 4.45 (m, 2H), 4.95 (d, 1H), 5.05 (d, 1H), 5.65 (m, 1H), 6.40 (d, 1H), 7.20 (d, 1H), 7.70 (m, 2H), 7.80 (m, 1H), 7.85 (br. s, 1H), 8.10 (br. d, 1H), 8.25 (d, 1H), 8.35 (s, 1H).

### Beispiel 4

### (rac)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure-allylester (XVI)

(*rac*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (IX) (500 g, 0.962 mol) wurde bei 20 °C in THF (2,5 l) vorgelegt und mit einer 1 M Lösung von Natriumhexamethyldisilazid (NaHMDS) in THF (203 g; 1,107 mol) versetzt. Nach 10 min Rühren wurde Dimethylsulfat (243 g; 1,925 mol) zugegeben und die Mischung für 2 h bei RT gerührt. Das Reaktionsgemisch wurde auf eine Lösung von 26%iger wässriger Ammoniaklösung (315 g; 4,812 mol) in 3 l Wasser gegeben und mit 250 ml THF nachgespült. Es wurde über Nacht nachgerührt, auf 5 °C abgekühlt. Das Produkt wurde abfiltriert und mit Wasser (1 l) nachgewaschen. Man trocknet bei 40°C im Vakuum.
Ausbeute: 443 g (86 % d. Theorie) eines beigefarbenen Feststoffes.
MS (ESIpos): *m*/*z* (%) = 534.1 (100) [M+H]⁺; MS (ESIneg): *m*/*z* (%) = 532.1 (100) [M-H]⁻.
¹H-NMR (400 MHz, DMSO-d₆): δ = 2.05 (s, 3H), 2.79 (s, 3H), 3.51 (s, 3H), 4.55 (m, 2H), 5,03 (d, 1H), 5.12 (d, 1H), 5.72 (m, 1H), 6.80 (s, 1H), 7.70 (m, 2H), 7.80 (m, 1H), 7.95 (br. s, 1H), 8.15 (br. d, 1H), 8.25 (d, 1H), 8.52 (s, 1H).

### Beispiel 5

### (rac)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure THF-Solvat (XXVI)

(*rac*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure-allylester (XVI) (485,6 g, 0.910 mol) wurde bei 20 °C in THF (2,275 l) vorgelegt und mit Morpholin (118,9 g; 1,365 mol) versetzt. Es wurde für 1 h Stickstoff in die Reaktionsmischung eingeleitet. Anschließend wurde auf 50 °C erwärmt, mit Palladium-(II)-acetat (511 mg; 2,275 mmol) und Triphenylphosphin (2388 mg; 9,102 mmol) versetzt und die Mischung für 2 h bei 50 °C gerührt. Nach Abkühlen wurde das Reaktionsgemisch in 4,5 1 Wasser gegeben. Es wurde mit 2 N Salzsäure auf pH = 2 eingestellt und das dabei entstehende Kristallisat über Nacht nachgerührt. Das Produkt wurde abfiltriert und mit Wasser (1.8 l) nachgewaschen. Es wurde bei 40°C im Vakuum getrocknet. Ausbeute: 504 g (98 % d. Theorie; bezogen auf das Mono-THF-Solvat) eines beigefarbenen Feststoffes.
MS (ESIpos): *m*/*z* (%) = 494.0 (100) [M+H]⁺.
¹H-NMR (400 MHz, DMSO-d₆): δ = 1.76 (m, 4 H; THF), 2.08 (s, 3H), 2.77 (s, 3H), 3.48 (s, 3H), 3,60 (m, 4H, THF), 6.72 (s, 1H), 7.75 (m, 2H), 7.82 (m, 1H), 7.92 (br. s, 1H), 8.11 (br. d, 1H), 8.27 (d, 1H), 8.46 (s, 1H), 12.75 (br. s, 1H).

### Beispiel 6

### (S)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure Chinidin Salz (XXVIII)

(*rac*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure THF-solvat (XXVI) (555 g, 0.910 mol) wurde bei 20 °C in Butylacetat (2,22 l) vorgelegt und mit (+)-Chinidin (334,3 g; 1,03 mol) versetzt. Anschließend wurde auf 50 °C erwärmt und 1 h bei 50 °C gerührt. Nach Abkühlen auf 5 °C wurde abfiltriert und der Filterkuchen mit Butylacetat (1.2 l) ausgerührt, erneut abfiltriert und mit Butylacetat (0,7 l) nachgewaschen. Es wurde bei 40°C im Vakuum getrocknet. Ausbeute: 361 g (45 % d. Theorie) eines cremefarbenen Feststoffes.
¹H-NMR (400 MHz, DMSO-d₆): δ = 1.58 (m, 2H), 1.79 (m, 1H), 2.04 (m, 1H), 2.07 (s, 3H), 2.33 (m, 1H), 2.77 (s, 3H), 2.79 (m, 1H), 2.90 (m, 2H), 3.21 (m, 1H), 3,33 (m, 2H), 3.51 (s, 3H), 3.90 (s, 3H), 5.11 (d, 1H), 5,14 (d, 1H), 5.53 (br. s, 1H), 6,09 (ddd, 1H), 6.72 (s, 1H), 7.75 (m, 2H), 7.82 (m, 1H), 7.92 (br. s, 1H), 8.11 (br. d, 1H), 8.27 (d, 1H), 8.46 (s, 1H), 12.75 (br. s, 1H).

### Beispiel 7

### (S)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure (XXVII)

(*S*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure Chinidin Salz (XXVIII) (360 g, 0.405 mol) wurde bei 60 °C in einer Mischung aus Wasser (3,9 l) und Isopropanol (0,4 l) suspendiert und mit 2 N Salzsäure auf pH = 1 gestellt und 1 h bei 60 °C gerührt. Nach Abkühlen auf 20 °C wurde abfiltriert, mit Wasser (0.6 l) nachgewaschen und der Filterkuchen mit Wasser (1.2 l) ausgerührt, erneut abfiltriert und mit Wasser (1,2 l) nachgewaschen. Es wurde bei 40°C im Vakuum getrocknet. Ausbeute: 196 g (92 % d. Theorie) eines cremefarbenen Feststoffes.
MS (ESIpos): *m*/*z* (%) = 494.0 (100) [M+H]⁺.
¹H-NMR (400 MHz, DMSO-d₆): δ = 2.08 (s, 3H), 2.77 (s, 3H), 3.48 (s, 3H), 6.72 (s, 1H), 7.75 (m, 2H), 7.82 (m, 1H), 7.92 (br. s, 1H), 8.11 (br. d, 1H), 8.27 (d, 1H), 8.46 (s, 1H), 12.75 (br. s, 1H).

### Beispiel 8

### (S)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbamid (XIX)

(*S*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure (XXVII) (390,5 g, 0.791 mol) wurde in THF (3,9 l) gelöst. Zur Entfernung von restlichen Wasserspuren wurden bei 80 °C Badtemperatur 2 l THF abdestilliert. Es wurde bei 0 °C mit 1,1-Carbonyldiimidazol (192,5 g, 1,187 mol) versetzt und 1 h bei 20 °C sowie 2 h bei 50 °C nachgerührt. Anschließend wurde bei 25 °C 26%ige wässrige Ammoniaklösung (518 g, 7,91 mol) zudosiert und 16 h nachgerührt. Die Reaktionsmischung wurde für 2 h auf 50 °C erwärmt, dabei wurde überschüssiger Ammoniak ausgegast. Nach Abkühlen wurde das Reaktionsgemisch in 7,8 l Wasser langsam zugegeben und das dabei entstehende Kristallisat über Nacht nachgerührt. Das Produkt wurde abfiltriert und mit Wasser (2,4 l) nachgewaschen. Man trocknet bei 40°C im Vakuum. Ausbeute: 361 g (92 % d. Theorie) eines cremefarbenen Feststoffes.
MS (ESIpos): *m*/*z* (%) = 493.0 (100) [M+H]⁺.
¹H-NMR (400 MHz, DMSO-d₆): δ = 1,73 (s, 3H), 2.73 (s, 3H), 3.45 (s, 3H), 6.55 (s, 1H), 7.34 (br. s, 1H), 7.48 (br. s, 1H), 7.73 (m, 2H), 7.80 (m, 2H), 8.11 (d, 1H), 8.43 (d, 1H), 8.47 (s, 1H).

### Beispiel 9

### (S)-4-[4-Cyano-2-(methylsulfonyl)phenyl] -3,6-dimethyl-2-oxo-1-[3-(trifluormethyl)phenyl]-1,2,3,4-tetrahydropyrimidin-5-carbonitril (I)

(*S*)-4-[4-Cyano-2-(methylsulfonyl)phenyl] -3,6-dimethyl-2-oxo-1-[3-(trifluormethyl)phenyl]-1,2,3,4-tetrahydropyrimidin-5-carbamid (XIX) (400 g, 0.812 mol) wurde in Ethylacetat (1,6 l) gelöst. Es wurde bei 20 °C mit N-Ethyldiisopropylamin (262,4 g, 2.031 mol) versetzt und 15 min bei 20 °C nachgerührt. Anschließend wurde bei 2 °C 26%ige eine 50%ige Lösung von 1-Propanphosponsäureanhydrid in Ethylacetat (1,137 kg, 1.79 mol) zudosiert, auf Rückflusstemperatur erwärmt und 2 h nachgerührt. Man ließ auf 20°C abkühlen und gab 3,4 l Wasser zum Ansatz. Nach Phasentrennung wurde die organische Phase mit gesättigter Natriumhydrogencarbonat-Lösung (1,2 l) gewaschen. Die organische Phase wurde auf 60 °C temperiert und unter gleichzeitiger Zugabe von Ethanol (Toluol vergällt) wurde im leichten Vakuum abdestilliert. Dabei kristallisiert das Produkt aus. Nach beendeter Kristallisation wurde auf Rückflusstemperatur erhitzt und 4 h nachgerührt. Es wurde auf 20°C abgekühlt und eine Stunde bei dieser Temperatur gerührt. Das Produkt wurde abfiltriert und einmal mit Wasser (1,2 l) und einmal mit Ethanol (Toluol vergällt) (0,4 l) nachgewaschen. Es wurde bei 50°C im Vakuum getrocknet. Ausbeute: 344 g (89 % d. Theorie) weiße Kristalle der stabilen Kristallform (A) mit einem Schmelzpunkt von 232 °C, Reinheit: 99,4 %, Gehalt: 99,3%
MS (ESIpos): *m*/*z* (%) = 475.1 (100) [M+H]⁺.
¹H-NMR (400 MHz, DMSO-d₆): δ = 1,81 (s, 3H), 2.70 (s, 3H), 3.52 (s, 3H), 6.48 (s, 1H), 7.65 - 8.40 (m, 6H), 8.46 (s, 1H).

### Beispiel 10

### (rac)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure (XXII)

(*rac*)-4-[4-Cyano-2-(methylsulfonyl)phenyl] -6-methyl-2-oxo-1-[3-(trifluormethyl)phenyl]-1,2,3,4-tetrahydropyrimidin-5-carbonsäure-allylester (IX) (420 g, 0.808 mol) wurde bei 20 °C in THF (2,1 l) vorgelegt und mit Morpholin (105,6 g; 1,213 mol) versetzt. Es wurde für 1 h Stickstoff in die Reaktionsmischung eingeleitet. Anschließend wurde mit Bis(triphenylphophin)palladium-(II)-chlorid (284 mg; 0,404 mmol) und Triphenylphosphin (424 mg; 1,617 mmol) versetzt und die Mischung für 2 h bei RT gerührt. Anschließend wurde nochmals mit Bis(triphenylphophin)palladium-(II)-chlorid (284 mg; 0,404 mmol) und Triphenylphosphin (424 mg; 1,617 mmol) versetzt und die Mischung für 2 h bei RT gerührt. Das Reaktionsgemisch wurde in 4 l Wasser gegeben. Es wurde mit 2 N Salzsäure auf pH = 2 eingestellt und das dabei entstehende Kristallisat über Nacht nachgerührt. Das Produkt wurde abfiltriert und mit Wasser (1,7 l) nachgewaschen. Es wurde bei 40°C im Vakuum getrocknet.
Ausbeute: 575 g (97 % d. Theorie) eines beigefarbenen Feststoffes.
MS (ESIpos): *m*/*z* (%) = 480.0 (100) [M+H]⁺.
¹H-NMR (400 MHz, DMSO-d₆): δ = 2.12 (s, 3H), 3.48 (s, 3H), 6.32 (s, 1H), 7,12 (s, 1H), 7.72 (m, 2H), 7.80 (m, 1H), 7.88 (br. s, 1H), 8.13 (br. d, 1H), 8.27 (d, 1H), 8.37 (s, 1H), 12.62 (br. s, 1H).

### Beispiel 11

### (S)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure Chinin Salz (XXIV)

(*rac*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure (XXII) (563,7 g, 1,176 mol) wurde bei 20 °C in einer Mischung aus Isopropanol/Wasser (9 : 1; 4,2 l) vorgelegt und mit (-)-Chinin (381,4 g; 1,176 mol) versetzt. Anschließend wurde auf 50 °C erwärmt und 1 h bei 50 °C gerührt. Nach Abkühlen auf 5 °C wurde abfiltriert und der Filterkuchen dreimal mit einerMischung aus Isopropanol/Wasser (9 : 1; 1,2 l) nachgewaschen. Es wurde bei 40°C im Vakuum getrocknet.
Ausbeute: 432 g (46 % d. Theorie) eines cremefarbenen Feststoffes.
¹H-NMR (400 MHz, DMSO-d₆): δ = 1.58 (m, 2H), 1.79 (m, 1H), 2.04 (m, 1H), 2.10 (s, 3H), 2.33 (m, 1H), 2.79 (m, 1H), 2.90 (m, 2H), 3.21 (m, 1H), 3,33 (m, 2H), 3.46 (s, 3H), 3.90 (s, 3H), 5.11 (d, 1H), 5,14 (d, 1H), 5.53 (br. s, 1H), 6,09 (m, 1H), 6.33 (s, 1H), 7.10 (s, 1H), 7.73 (m, 2H), 7.82 (m, 1H), 7.90 (br. s, 1H), 8.11 (br. d, 1H), 8.27 (d, 1H), 8.44 (s, 1H), 12.70 (br. s, 1H).

### Beispiel 12

### (S)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure (XI)

(*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure Chinidin Salz (XXIV) (430 g, 0,535 mol) wurde bei 60 °C in einer Mischung aus Wasser (4,2 l) und Isopropanol (0,4 l) suspendiert und mit 2 N Salzsäure auf pH = 1 gestellt und 1 h bei 60 °C gerührt. Nach Abkühlen auf 20 °C wurde abfiltriert und der Filterkuchen dreimal mit Wasser (0,6 l) nachgewaschen. Es wurde bei 40°C im Vakuum getrocknet.
Ausbeute: 251 g (98 % d. Theorie) eines cremefarbenen Feststoffes.
MS (ESIpos): *m*/*z* (%) = 480.0 (100) [M+H]⁺.
¹H-NMR (400 MHz, DMSO-d₆): δ = 2.12 (s, 3H), 3.48 (s, 3H), 6.32 (s, 1H), 7,12 (s, 1H), 7.72 (m, 2H), 7.80 (m, 1H), 7.88 (br. s, 1H), 8.13 (br. d, 1H), 8.27 (d, 1H), 8.37 (s, 1H), 12.62 (br. s, 1H).

### Beispiel 13

### (S)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (X)

(*S*)-4- [4-Cyano-2-(methylsulfonyl)phenyl]-6-methyl-2-oxo-1-[3-(trifluormethyl)phenyl]-1,2,3,4-tetrahydropyrimidin-5-carbonsäure (XI) (250 g, 0.521 mol) wurde bei 20 °C in Aceton (1,5 l) vorgelegt und mit Kaliumcarbonat (72 g; 0,521 mol) versetzt. Nach 10 min Rühren wurde Allylbromid (79 g; 652 mol) zugegeben und die Mischung für 6 h unter Rückfluss gerührt. Nach Abkühlen wurden zum Reaktionsgemisch 1,4 l Wasser zugegeben und es wurde 60 min nachgerührt. Das Produkt wurde abfiltriert, zweimal mit Wasser (0,6 l) und zweimal mit MtBE (0,6 l) nachgewaschen. Es wurde bei 40°C im Vakuum getrocknet.
Ausbeute: 258 g (95 % d. Theorie) eines cremefarbenen Feststoffes.
MS (ESIpos): *m*/*z* (%) = 534.1 (100) [M+H]⁺.
¹H-NMR (400 MHz, DMSO-d₆): δ = 2.05 (s, 3H), 2.79 (s, 3H), 3.51 (s, 3H), 4.55 (m, 2H), 5,03 (d, 1H), 5.12 (d, 1H), 5.72 (m, 1H), 6.80 (s, 1H), 7.70 (m, 2H), 7.80 (m, 1H), 7.95 (br. s, 1H), 8.15 (br. d, 1H), 8.25 (d, 1H), 8.52 (s, 1H).

### Beispiel 14

### (S)-4-[4-Cyano-2-(methylsulfonyl)phenyl] -3,6-dimethyl-2-oxo-1-[3-(trifluormethyl)phenyl]-1,2,3,4-tetrahydropyrimidin-5-carbonsäure-allylester (XXIII)

(*S*)-4- [4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (X) (250 g, 0.481 mol) wurde bei 20 °C in THF (1,25 l) vorgelegt und mit einer 1 M Lösung von Natriumhexamethyldisilazid (NaHMDS) in THF (102 g; 0,554 mol) versetzt. Nach 10 min Rühren wurde Dimethylsulfat (122 g; 0,964 mol) zugegeben und die Mischung für 2 h bei RT gerührt. Das Reaktionsgemisch wurde auf eine Lösung von 26%iger wässriger Ammoniaklösung (178 g; 2,4 mol) in 1,5 l Wasser gegeben und mit 200 ml THF nachgespült. Es wurde über Nacht nachgerührt, auf 5 °C abgekühlt. Das Produkt wird abfiltriert und mit Wasser (0,6 l) nachgewaschen. Es wurde bei 40°C im Vakuum getrocknet.
Ausbeute: 230 g (89 % d. Theorie) eines beigefarbenen Feststoffes.
MS (ESIpos): *m*/*z* (%) = 534.1 (100) [M+H]⁺.
¹H-NMR (400 MHz, DMSO-d₆): δ = 2.05 (s, 3H), 2.79 (s, 3H), 3.51 (s, 3H), 4.55 (m, 2H), 5,03 (d, 1H), 5.12 (d, 1H), 5.72 (m, 1H), 6.80 (s, 1H), 7.70 (m, 2H), 7.80 (m, 1H), 7.95 (br. s, 1H), 8.15 (br. d, 1H), 8.25 (d, 1H), 8.52 (s, 1H).

### Beispiel 15

### (R)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure (XXX)

Die gesammelten Mutterlaugen und Waschlaugen aus Beispiel 11 welche das Cininsalz der (*R*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure (XXV) enthalten wurden bis zum Kristallbrei eingeengt. Es wurde in Wasser (5,0 l) aufgenommen, mit 2 N Salzsäure auf pH = 1 gestellt und 1 h bei 60 °C gerührt. Nach Abkühlen auf 20 °C wurde abfiltriert und der Filterkuchen dreimal mit Wasser (1.0 l) nachgewaschen. Es wurde bei 40°C im Vakuum getrocknet.
Ausbeute: 293 g (52 % d. Theorie, bezogen auf Einsatz XXII) eines cremefarbenen Feststoffes.
Verhältnis R-Enantiomer : S-Enantiomer: 88 : 12
MS (ESIpos): *m*/*z* (%) = 480.0 (100) [M+H]⁺.
¹H-NMR (400 MHz, DMSO-d₆): δ = 2.12 (s, 3H), 3.48 (s, 3H), 6.32 (s, 1H), 7,12 (s, 1H), 7.72 (m, 2H), 7.80 (m, 1H), 7.88 (br. s, 1H), 8.13 (br. d, 1H), 8.27 (d, 1H), 8.37 (s, 1H), 12.62 (br. s, 1H).

### Beispiel 16

### (R)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (XXXI)

(*R*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure (XXX) (292 g, 0,609 mol) wurde bei 20 °C in Aceton (1,6 l) vorgelegt und mit Kaliumcarbonat (84 g; 0,609 mol) versetzt. Nach 10 min Rühren wurde Allylbromid (92 g; 761 mol) zugegeben und die Mischung für 6 h unter Rückfluss gerührt. Nach abkühlen wurden zum Reaktionsgemisch 1,5 l Wasser zugegeben und wurde 60 min nachgerührt. Das Produkt wird abfiltriert, zweimal mit Wasser (0,6 l) und zweimal mit MtBE (0,6 l) nachgewaschen. Man trocknet bei 40°C im Vakuum.
Ausbeute: 301 g (95 % d. Theorie) eines cremefarbenen Feststoffes.
MS (ESIpos): *m*/*z* (%) = 534.1 (100) [M+H]⁺.
¹H-NMR (400 MHz, DMSO-d₆): δ = 2.05 (s, 3H), 2.79 (s, 3H), 3.51 (s, 3H), 4.55 (m, 2H), 5,03 (d, 1H), 5.12 (d, 1H), 5.72 (m, 1H), 6.80 (s, 1H), 7.70 (m, 2H), 7.80 (m, 1H), 7.95 (br. s, 1H), 8.15 (br. d, 1H), 8.25 (d, 1H), 8.52 (s, 1H).

### Beispiel 17

### Isomerisierung von (R)-4-[4-Cyano-2-(methylsulfonyl)phenyl]-6-methyl-2-oxo-1-[3-(trifluormethyl)-phenyl]-1,2,3,4-tetrahydropyrimidin-5-carbonsäure-allylester (XXXI) zum (rac)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (IX)

(*R*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure (XXXI) (292 g, 0.609 mol) wurde bei 20 °C in Aceton (1,6 l) vorgelegt und mit Kaliumcarbonat (84 g; 0,609 mol) versetzt. Nach 10 min Rühren wurde Allylbromid (92 g; 761 mol) zugegeben und die Mischung für 6 h unter Rückfluss gerührt. Nach Abkühlen wurden zum Reaktionsgemisch 1,5 l Wasser zugegeben und wurde 60 min nachgerührt. Das Produkt wurde abfiltriert, zweimal mit Wasser (0,6 l) und zweimal mit MtBE (0,6 l) nachgewaschen. Es wurde bei 40°C im Vakuum getrocknet.
Ausbeute: 301 g (95 % d. Theorie) eines cremefarbenen Feststoffes.
MS (ESIpos): *m*/*z* (%) = 534.1 (100) [M+H]⁺.
¹H-NMR (400 MHz, DMSO-d₆): δ = 2.05 (s, 3H), 2.79 (s, 3H), 3.51 (s, 3H), 4.55 (m, 2H), 5,03 (d, 1H), 5.12 (d, 1H), 5.72 (m, 1H), 6.80 (s, 1H), 7.70 (m, 2H), 7.80 (m, 1H), 7.95 (br. s, 1H), 8.15 (br. d, 1H), 8.25 (d, 1H), 8.52 (s, 1H).

### Beispiel 18

### Physikochemische Charakterisierung von Verbindung der Formel (I) in der Kristallform (A)

### Parameter der Röntgendiffraktometrie für die Messung der Verbindung der Formel (I) in der Kristallform (A):

Gerät: Transmission diffractometer PANalytical X'Pert PRO with PIXcel counter (multichannel)

| | |
|---|---|
| Scan-Achse | 2Theta-Omega |
| Startposition [°2Th.] | 2,0000 |
| Endposition [°2Th.] | 37,9900 |
| Art der Divergenzblende | Fest |
| Größe der Divergenzblende [°] | 1,0000 |
| Temperatur der Messung [°C] | 25 |
| Anodenmaterial | Cu |
| K-Alphal [Å] | 1,54060 |
| Generatoreinstellung | 40 mA, 40 kV |
| Diffraktometer Typ | Transmissions Diffraktometer |
| Goniometer Radius [mm] | 240,00 |
| Abstand Focus-Div.blende [mm] | 91,00 |
| Primärstrahl- Monochromator | fokussierender Röntgenspiegel |
| Probendrehung | Ja |

**Tabelle 1: Peakmaxima [2 Theta] des Röntgendiffraktogramms der Verbindung (I) in der Kristallform (A)**

| **Peakmaximum [2 Theta]** | | |
|---|---|---|
| **Verbindung (I), Kristallform (A)** | | |
| 7.5 | 20.0 | 28.0 |
| 10.0 | 20.8 | 28.1 |
| 11.5 | 20.9 | 28.3 |
| 11.9 | 21.8 | 28.7 |
| 12.2 | 22.5 | 29.2 |
| 12.4 | 22.9 | 29.6 |
| 13.2 | 23.1 | 30.3 |
| 14.7 | 23.4 | 30.5 |
| 15.1 | 23.5 | 30.8 |
| 15.8 | 24.0 | 31.7 |
| 16.0 | 24.7 | 32.2 |
| 16.5 | 25.1 | 32.4 |
| 17.8 | 25.3 | 33.4 |
| 18.5 | 25.6 | 33.8 |
| 18.7 | 26.5 | 34.2 |
| 19.4 | 27.1 | 34.5 |
| 19.8 | 27.4 | |

### Messbedingungen für die Ramanspektroskopie für die Messung der Verbindung der Formel (I) in der Kristallform (A):

| Gerät | Bruker Raman RFS 100/S |
|---|---|
| Anzahl Scans | 64 |
| Auflösung | 2 - 4 cm⁻¹ |
| Laser Power | 50 mW |
| Laser Wellenlänge | 1064 nm |

**Tabelle 2: Bandenmaxima des Raman Spektrums der Verbindung (I) in der Kristallform (A)**

| **Bandenmaximum [cm-1]** | | |
|---|---|---|
| **Modifikation I** | | |
| 3087 | 1312 | 589 |
| 3075 | 1299 | 580 |
| 3067 | 1238 | 535 |
| 3044 | 1195 | 490 |
| 3019 | 1169 | 471 |
| 2993 | 1154 | 457 |
| 2969 | 1142 | 443 |
| 2928 | 1091 | 435 |
| 2918 | 1077 | 403 |
| 2236 | 1066 | 365 |
| 2216 | 1056 | 346 |
| 2184 | 1015 | 329 |
| 1646 | 1004 | 298 |
| 1605 | 994 | 280 |
| 1443 | 910 | 255 |
| 1435 | 873 | 240 |
| 1418 | 795 | 217 |
| 1411 | 767 | 190 |
| 1395 | 761 | 171 |
| 1387 | 746 | 149 |
| 1361 | 683 | 128 |
| 1354 | 674 | 111 |
| 1331 | 645 | |

### Beschreibung der Abbildungen:

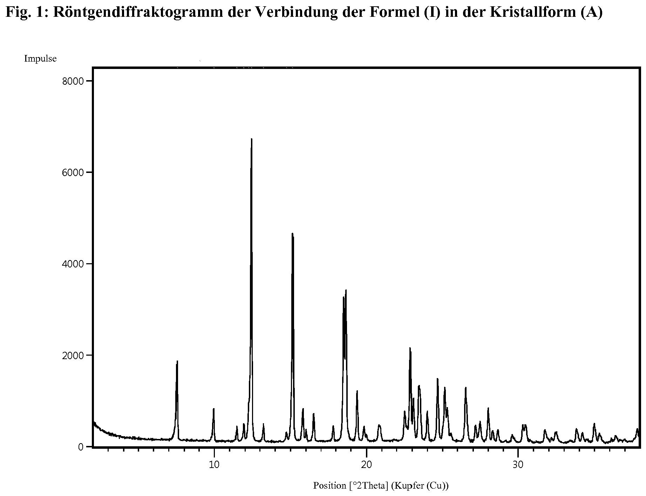

**Abbildung 1****:** Röntgendiffraktrogramm derVerbindung der Formel (I) in der Kristallform (A)

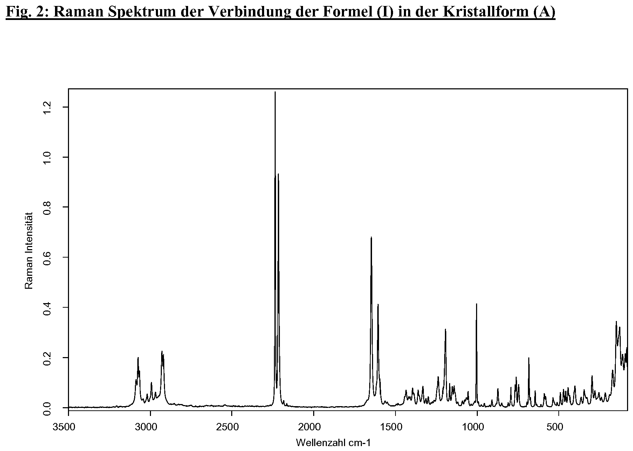

**Abbildung 2****:** Raman Spektrum der Verbindung der Formel (I) in der Kristallform (A)

## Patentansprüche

1. Verbindung der Formel (I) 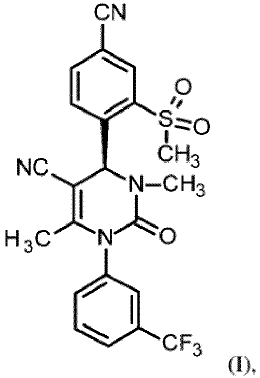 in der Kristallform (A), **dadurch gekennzeichnet, dass** das Röntgendiffraktrogramm der Verbindung Peakmaxima des 2 Theta Winkels bei 7.5, 12.4, 15.1, 18.5, 18.7, 22.9, 24.7 und 26.5 zeigt.

2. Verbindung der Formel (I) in der Kristallform (A) nach Anspruch 1, **dadurch gekennzeichnet, dass** das Raman- Spektrum der Verbindung Bandenmaxima bei 3075, 2928, 2918, 2236, 2216, 1646, 1605, 1195 und 1004 cm⁻¹ zeigt.

3. Verfahren zur Herstellung der Verbindung der Formel (I) in der Kristallform (A) nach Anspruch 1 oder 2, **dadurch gekennzeichnet, dass** eine Verbindung der Formel (I), vorliegend in einer oder mehreren Kristallformen oder als Solvat, in einem Alkohol, bevorzugt Ethanol, auskristallisiert wird, anschließend der dabei entstehende Kristallbrei auf 50-80°C erhitzt und 2-5 h bei dieser Temperatur nachgerührt wird.

4. Verbindung nach einem der Ansprüche 1 oder 2 zur Verwendung in einem Verfahren zur Behandlung von Krankheiten.

5. Verbindung nach einem der Ansprüche 1 oder 2 zur Verwendung in einem Verfahren zur Behandlung und/oder Prävention von Erkrankungen der Lunge und des Herz-/Kreislaufsystems und zur Förderung der Wundheilung, insbesondere bei chronischen Wunden.

6. Verbindung nach einem der Ansprüche 1 oder 2 zur Verwendung in einem Verfahren zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD), der zystischen Fibrose (CF), der Bronchiektasie und zur Förderung der Wundheilung, insbesondere bei chronischen Wunden.

7. Arzneimittel enthaltend die Verbindung der Formel (I) in der Kristallform (A) gemäß einem der Ansprüche 1 oder 2 in mehr als 90 Gewichtsprozente bezogen auf die Gesamtmenge der enthaltenen Verbindung der Formel (I).

8. Verwendung der Verbindung der Formel (I) in der Kristallform (A) gemäß einem der Ansprüche 1 oder 2 zur Herstellung eines Arzneimittels zur Behandlung von Erkrankungen der Lunge und des Herz-/Kreislaufsystems und zur Förderung der Wundheilung, insbesondere bei chronischen Wunden.

9. Verfahren gemäß Anspruch 3 zur Herstellung einer Verbindung der Formel (I) in der Kristallform (A) nach einem der Ansprüche 1 oder 2 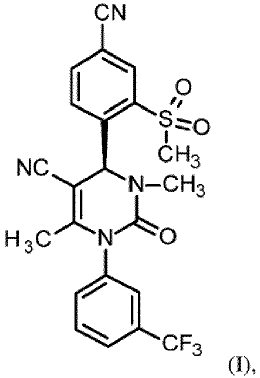 **dadurch gekennzeichnet, dass** man eine Verbindung der Formel (IX) 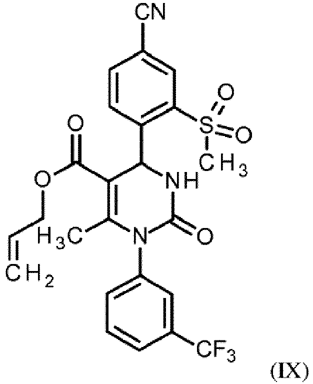
a-1) in Gegenwart eines Methylierungsmittels und einer Base zu einer Verbindung der Formel (XVI) 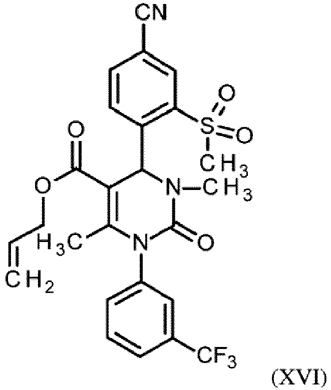 umsetzt; anschließend
a-2) eine Verbindung der Formel (XVI) in Gegenwart eines Palladiumkatalysators und einer sekundären Amin-Base zu einer Verbindung der Formel (XXVI) 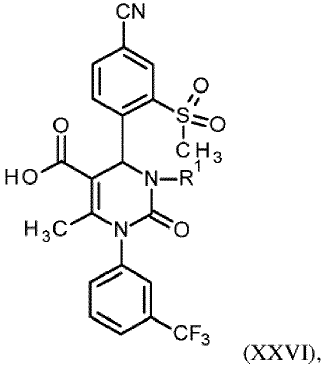 umsetzt, in welcher R¹ für Methyl steht,
oder
b-1) in Gegenwart eines Palladiumkatalysators und einer sekundären Amin-Base zu einer Verbindung der Formel (XXVI) umsetzt, in welcher R¹ für Wasserstoff steht;
anschließend
c) eine Verbindung der Formel (XXVI), in welcher R¹ für Wasserstoff oder Methyl steht, in Gegenwart eines China-Alkaloids und eines Lösungsmittels zu Verbindungen der Formeln (XXVIII) und (XXIX) 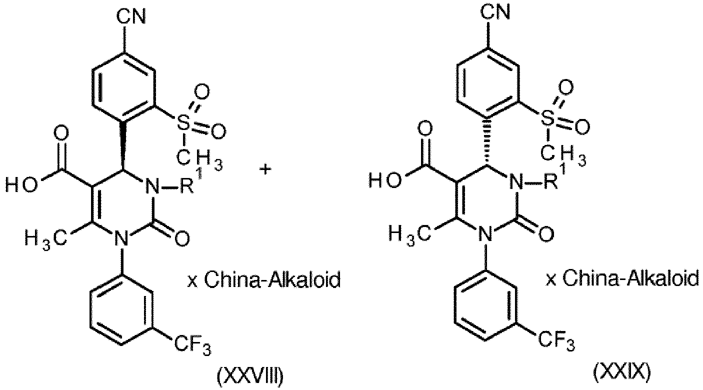 umsetzt, in welchen R¹ in Formel (XXVIII) und in Formel (XXIX) für Wasserstoff steht oder in welchen R¹ in Formel (XXVIII) und in Formel (XXIX) für Methyl steht; anschließend
d) eine Verbindung der Formel (XXVIII) isoliert; anschließend
e) eine Verbindung der Formel (XXVIII) in Gegenwart einer starken Säure zu einer Verbindung der Formel (XXVII) 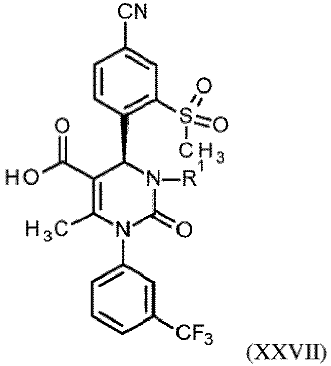 umsetzt, in welcher R¹ für Wasserstoff oder Methyl steht; anschließend
b-2) für den Fall, dass R¹ in der Verbindung der Formel (XXVII) für Wasserstoff steht, eine Verbindung der Formel (XXVII) in Gegenwart eines Allylhalogenids oder -sulfonats und einer Base zu einer Verbindung der Formel (X) 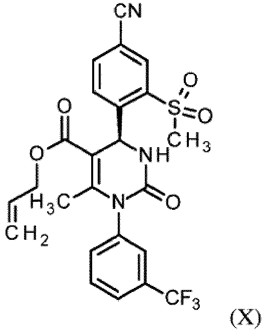 umsetzt, anschließend
b-3) eine Verbindung der Formel (X) in Gegenwart eines Methylierungsmittels und einer Base zu einer Verbindung der Formel (XXIII) 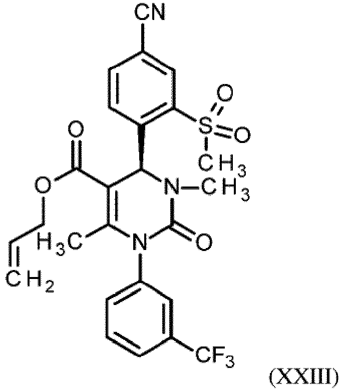 umsetzt, anschließend
b-4) eine Verbindung der Formel (XXIII) in Gegenwart eines Palladiumkatalysators und einer sekundären Amin-Base zu einer Verbindung der Formel (XXVII) 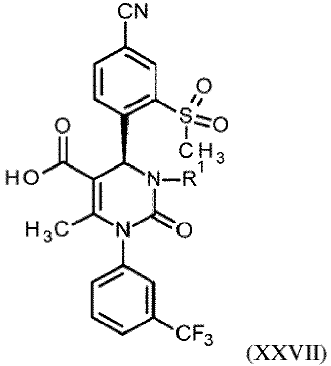 umsetzt, in welcher R¹ für Methyl steht; anschließend
f) eine Verbindung der Formel (XXVII), in welcher R¹ für Methyl steht, in Gegenwart eines Aktivierungsreagenzes zu einer Verbindung der Formel (XIX) 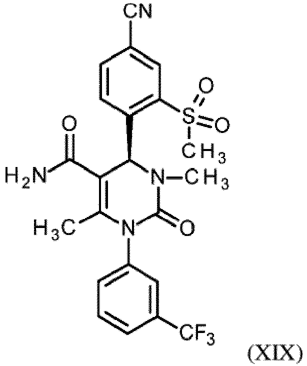 umsetzt, anschließend
g) eine Verbindung der Formel (XIX) in Gegenwart eines Dehydratisierungsmittels zu einer Verbindung der Formel (I) einsetzt;
gegebenenfalls im Anschluss an Reaktionsschritt c) eine Verbindung der Formel (XXIX) isoliert 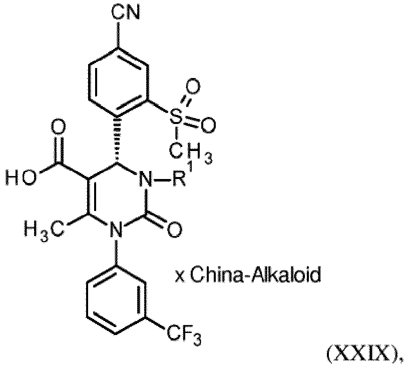 in der R¹ für Wasserstoff steht, diese gemäß Reaktionsschritt e) in Gegenwart einer starken Säure zu einer Verbindung der Formel (XXX) 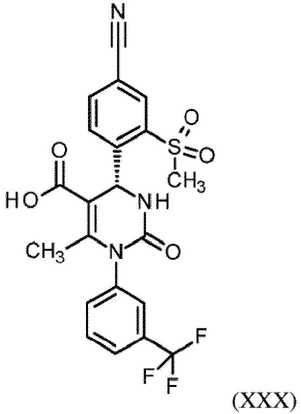 umsetzt; anschließend eine Verbindung der Formel (XXX) gemäß Reaktionsschritt b-2) in Gegenwart eines Allylhalogenids oder -sulfonats und einer Base zu einer Verbindung der Formel (XXXI) 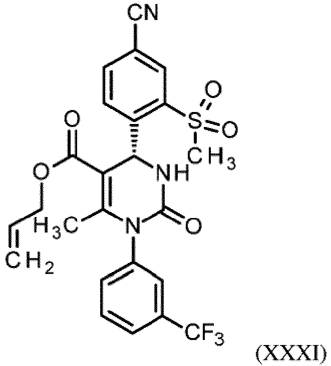 umsetzt; anschließend
h) eine Verbindung der Formel (XXXI) in Gegenwart einer starken, nicht nukleophilen Base in einem Lösungsmittel und unter gleichzeitigem Erwärmen in das Racemat der Formel (IX) 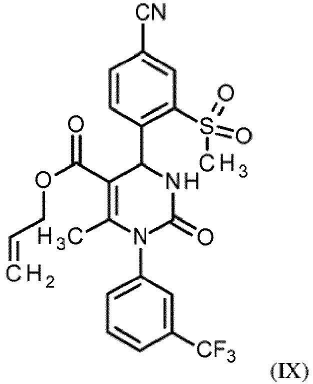 umsetzt; anschließend eine Verbindung der Formel (IX) gemäß den oben beschriebenen Reaktionsschritten b-1), c), d), e), b-2), b-3), b-4), f) und g) zu einer Verbindung der Formel (I) umsetzt;
und gegebenenfalls die Reaktionsschritte der Isolierung einer Verbindung der Formel (XXIX) deren Umsetzung gemäß Reaktionsschritt e) in Gegenwart einer starken Säure zu einer Verbindung der Formel (XXX), die anschließende Umsetzung einer Verbindung der Formel (XXX) gemäß Reaktionsschritt b-2) in Gegenwart eines Allylhalogenids oder -sulfonats und einer Base zu einer Verbindung der Formel (XXXI), und anschließende Durchführung der Reaktionsschritte h), b-1), c), d), e), b-2), b-3), b-4), f) und g) einmal oder mehrmals wiederholt;
wobei gegebenenfalls in den Formeln (XXVI), (XXVII), (XXVIII) und (XXIX) R¹ für Methyl steht und das Verfahren die Reaktionsschritte a-1), a-2), c), d), e), f) und g) umfasst;
wobei gegebenenfalls in den Formeln (XXVI), (XXVII), (XXVIII) und (XXIX) R¹ für Wasserstoff steht und das Verfahren die Reaktionsschritte b-1), c), d), e), b-2), b-3), b-4), f) und g) umfasst;
gegebenenfalls **dadurch gekennzeichnet, dass** man
im Anschluss an Reaktionsschritt c) eine Verbindung der Formel (XXIX) isoliert, diese gemäß Reaktionsschritt e) in Gegenwart einer starken Säure zu einer Verbindung der Formel (XXX) umsetzt; anschließend eine Verbindung der Formel (XXX) gemäß Reaktionsschritt b-2) in Gegenwart eines Allylhalogenids oder -sulfonats und einer Base zu einer Verbindung der Formel (XXXI) umsetzt; anschließend
h) eine Verbindung der Formel (XXXI) in Gegenwart einer starken, nicht nukleophilen Base in einem Lösungsmittel und unter gleichzeitigem Erwärmen in das Racemat der Formel (IX) umsetzt; anschließend
eine Verbindung der Formel (IX) gemäß den oben beschriebenen Reaktionsschritten b-1), c), d), e), b-2), b-3), b-4), f) und g) zu einer Verbindung der Formel (I) umsetzt;
und gegebenenfalls die Reaktionsschritte der Isolierung einer Verbindung der Formel (XXIX) deren Umsetzung gemäß Reaktionsschritt e) in Gegenwart einer starken Säure zu einer Verbindung der Formel (XXX), die anschließende Umsetzung einer Verbindung der Formel (XXX) gemäß Reaktionsschritt b-2) in Gegenwart eines Allylhalogenids oder -sulfonats und einer Base zu einer Verbindung der Formel (XXXI), und anschließende Durchführung der Reaktionsschritte h), b-1), c), d), e), b-2), b-3), b-4), f) und g) einmal oder mehrmals wiederholt
wobei man die Verbindung der Formel (I) aus der organischen Phase aus Reaktionsschritt g) in einem Alkohol, bevorzugt Ethanol, auskristallisiert, anschließend den dabei entstehenden Kristallbrei auf 50-80°C erhitzt und 2-5 h bei dieser Temperatur nachrührt.

## Claims

1. A compound of formula (I) 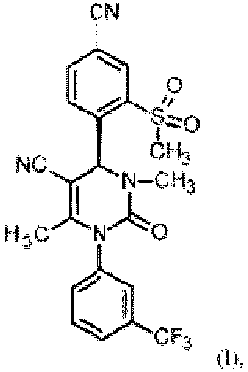 in the crystal form (A), **characterized in that** the X-ray diffraction pattern of the compound shows peak maxima of the 2 theta angle at 7.5, 12.4, 15.1, 18.5, 18.7, 22.9, 24.7 and 26.5.

2. The compound of formula (I) in the crystal form (A) according to claim 1, **characterized in that** the Raman spectrum of the compound shows band maxima at 3075, 2928, 2918, 2236, 2216, 1646, 1605, 1195 and 1004 cm⁻¹.

3. A method for preparing the compound of formula (I) in the crystal form (A) according to any one of claims 1 or 2, **characterized in** crystallizing a compound of formula (I), present in one or more crystal forms or as a solvate, from an alcohol, preferably ethanol, and subsequently heating the resulting crystal paste to 50-80°C with stirring for 2-5 h at this temperature.

4. The compound according to any one of claims 1 or 2 for use in a method for the treatment of illnesses.

5. The compound according to any one of claims 1 or 2 for use in a method for the treatment and/or prevention of diseases of the lung and the cardiovascular system and for promoting wound healing, in particular of chronic wounds.

6. A compound according to any one of claims 1 or 2 for use in a method for the treatment and/or prevention of pulmonary arterial hypertonia (PAH) and other forms pulmonary hypertonia (PH), chronic obstructive lung diseases (COPD), acute lung injury (ALI), acute respiratory disease syndrome (ARDS), pulmonary emphysema, alpha l-antitrypsin deficiency (AATD), cystic fibrosis (CF), bronchiectasis and for promoting wound healing, in particular of chronic wounds.

7. A pharmaceutical composition comprising the compound of formula (I) in the crystal form (A) according to any one of claims 1 or 2 in more than 90 wt. percent of the total quantity of the compound of formula (I) contained therein.

8. Use of the compound of formula (I) in the crystal form (A) according to any one of claims 1 or 2 for preparing a pharmaceutical composition for the treatment of diseases of the lung and the cardiovascular system and for promoting wound healing, in particular of chronic wounds.

9. A method according to claim 3 for preparing a compound of formula (I) in the crystal form (A) according to any one of claims 1 or 2 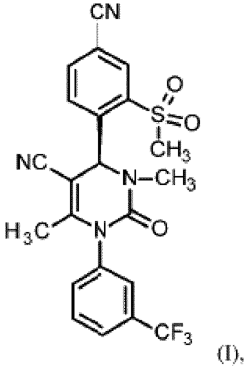 **characterized in** reacting a compound of formula (IX) 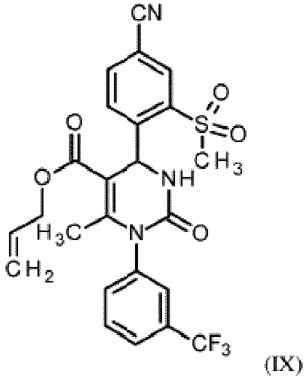
a-1) in the presence of a methylation agent and a base to form a compound of formula (XVI) 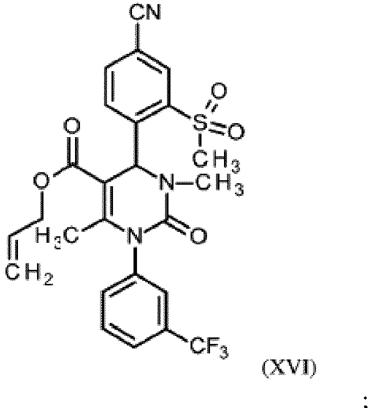 then
a-2) reacting a compound of formula (XVI) in the presence of a palladium catalyst and a secondary amine base to form a compound of formula (XXVI) 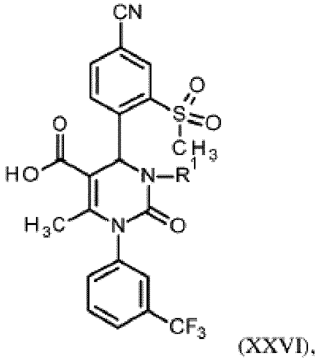 wherein R¹ is methyl,
or
b-1) reacting in the presence of a palladium catalyst and a secondary amine base to form a compound of formula (XXVI), wherein R¹ is hydrogen;
then
c) reacting a compound of formula (XXVI), wherein R¹ is hydrogen or methyl, in the presence of a cinchona alkaloid and a solvent to form compounds of formulas (XXVIII) and (XXIX) 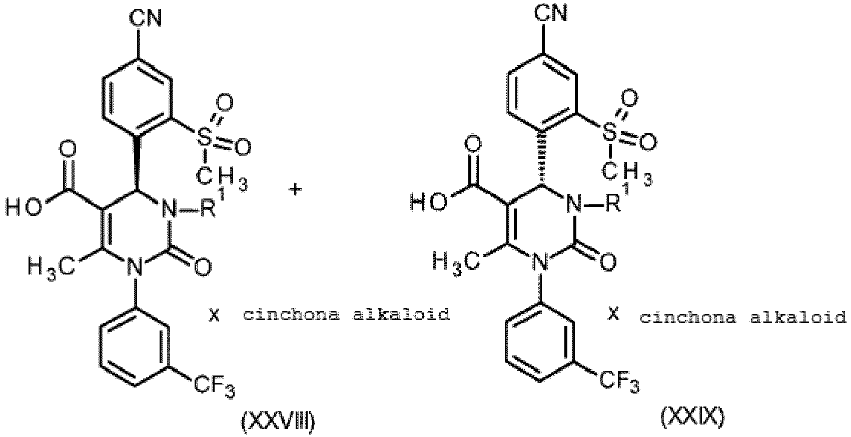 wherein R¹ in formula (XXVIII) and in formula (XXIX) is hydrogen or wherein R¹ in formula (XXVIII) and in formula (XXIX) is methyl; then
d) isolating a compound of formula (XXVIII); then
e) reacting a compound of formula (XXVIII) in the presence of a strong acid to form a compound of formula (XXVII) 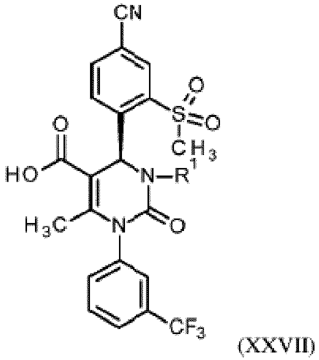 wherein R¹ is hydrogen or methyl; then
b-2) if R¹ in the compound of formula (XXVII) is hydrogen, reacting a compound of formula (XXVII) in the presence of an allyl halide or -sulfonate and a base to form a compound of formula (X) then 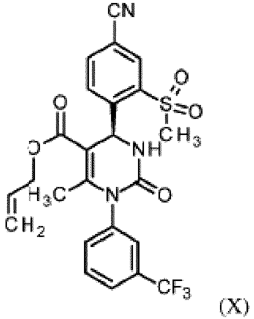
b-3) reacting a compound of formula (X) in the presence of a methylation agent and a base to form a compound of formula (XXIII) 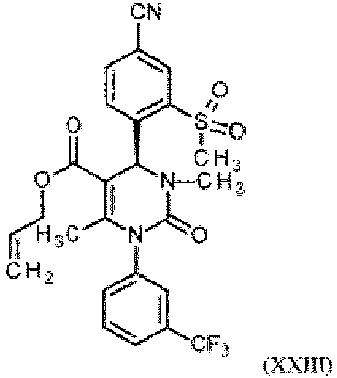 then
b-4) reacting a compound of formula (XXIII) in the presence of a palladium catalyst and a secondary amine base to form a compound of formula (XXVII) 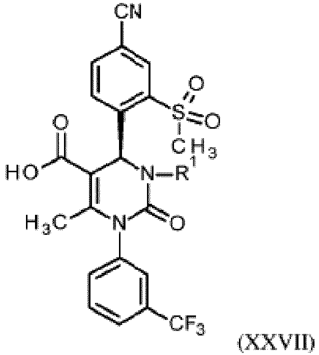 wherein R¹ is methyl; then
f) reacting a compound of formula (XXVII), wherein R¹ is methyl, in the presence of an activation reagent, to form a compound of formula (XIX) 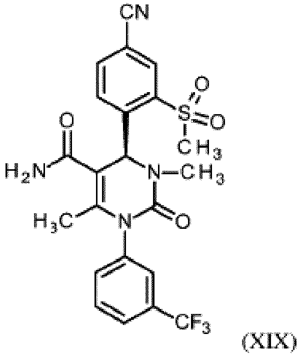 then
g) reacting a compound of formula (XIX) in the presence of a dehydrating agent, to provide a compound of formula (I);
and, optionally, after reaction step c), isolating a compound of formula (XXIX) 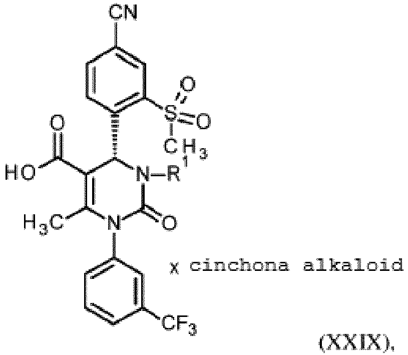 wherein R¹ is hydrogen, reacting said compound according to reaction step e) in the presence of a strong acid to form a compound of formula (XXX) 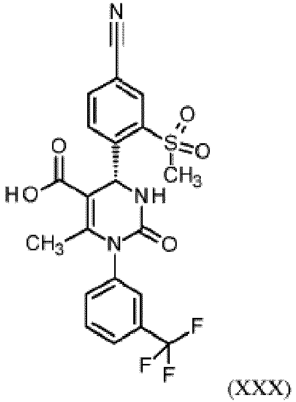 then reacting a compound of formula (XXX) according to reaction step b-2) in the presence of an allyl halide or -sulfonate and a base to form a compound of formula (XXXI) then 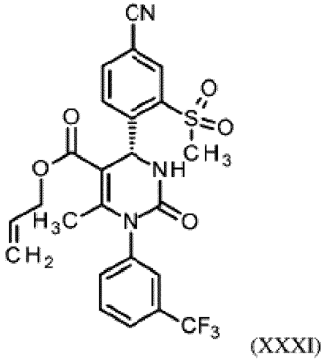
h) reacting a compound of formula (XXXI) in the presence of a strong, non-nucleophilic base in a solvent and under simultaneous heating to form the racemate of formula (IX) 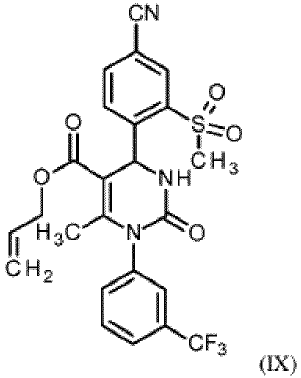 then reacting a compound of formula (IX) according to the above-described reaction steps b-1), c), d), e), b-2), b-3), b-4), f) and g) to form the compound of formula (I);
and, optionally, repeating one or more times the reaction steps of isolating a compound of formula (XXIX), its reaction according to reaction step e) in the presence of a strong acid to form a compound of formula (XXX), the subsequent reaction of a compound of formula (XXX) according to reaction step b-2) in the presence of an allyl halide or -sulfonate and a base to form a compound of formula (XXXI), and subsequently performing reaction steps h), b-1), c), d), e), b-2), b-3), b-4), f) and g);
wherein, optionally, R¹ in formulas (XXVI), (XXVII), (XXVIII) and (XXIX) is methyl and the method comprises the reaction steps a-1), a-2), c), d), e), f) and g);
wherein, optionally, R¹ in formulas (XXVI), (XXVII), (XXVIII) and (XXIX) is hydrogen and the method comprises the reaction steps b-1), c), d), e), b-2), b-3), b-4), f) and g);
optionally, **characterized in that**,
after reaction step c), a compound of formula (XXIX) is isolated; said compound is reacted according to reaction step e) in the presence of a strong acid to form a compound of formula (XXX); then, a compound of formula (XXX) is reacted according to reaction step b-2) in the presence of an allyl halide or -sulfonate and a base to form a compound of formula (XXXI); then
h) reacting a compound of formula (XXXI) in the presence of a strong, non-nucleophilic base in a solvent and under simultaneous heating to form the racemate of formula (IX); then
reacting a compound of formula (IX) according to the above-described reaction steps b-1), c), d), e), b-2), b-3), b-4), f) and g) to form a compound of formula (I);
and, optionally, repeating one or more times the reaction steps of isolating a compound of formula (XXIX), its reaction according to reaction step e) in the presence of a strong acid to form a compound of formula (XXX), the subsequent reaction of a compound of formula (XXX) according to reaction step b-2) in the presence of an allyl halide or -sulfonate and a base to form a compound of formula (XXXI), and subsequently performing reaction steps h), b-1), c), d), e), b-2), b-3), b-4), f) and g);
wherein the compound of formula (I) is crystallized from the organic phase of reaction step g) from an alcohol, preferably ethanol, and then the resulting crystal paste is heated to 50-80°C, and stirred for 2-5 h at this temperature.

## Revendications

1. Composé répondant à la formule (I) 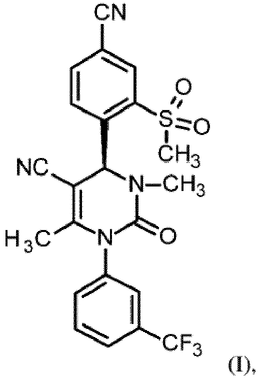 sous la forme cristalline (A), **caractérisé en ce que** le diffractogramme aux rayons X du composé montre des maxima de pic de l'angle 2-thêta à 7.5, 12.4, 15.1, 18.5, 18.7, 22.9, 24.7 et 26.5.

2. Composé répondant à la formule (I) sous la forme cristalline (A) selon la revendication 1, **caractérisé en ce que** le spectre Raman du composé montre des maxima de bande à 3075, 2928, 2918, 2236, 2216, 1646, 1605, 1195 et 1004 cm⁻¹.

3. Procédé de production du composé répondant à la formule (I) sous la forme cristalline (A) selon la revendication 1 ou 2, **caractérisé en ce qu'**un composé répondant à la formule (I), présent sous une ou plusieurs formes cristallines ou en tant que solvate, est mis à cristalliser dans un alcool, de préférence l'éthanol, après quoi la masse cristalline qui en résulte est chauffée entre 50 et 80 °C et agitée pendant 2 à 5 h à cette température.

4. Composé selon l'une des revendications 1 et 2 destiné à être utilisé dans un procédé de traitement de maladies.

5. Composé selon l'une des revendications 1 et 2 destiné à être utilisé dans un procédé de traitement et/ou de prévention de troubles pulmonaires et du système cardiaque/circulatoire et permettant de favoriser la cicatrisation, notamment dans le cas de plaies chroniques.

6. Composé selon l'une des revendications 1 et 2 destiné à être utilisé dans un procédé de traitement et/ou de prévention de l'hypertension artérielle pulmonaire (HTAP) et d'autres formes d'hypertension pulmonaire (HTP), des bronchopneumopathies chroniques obstructives (BPCO), de la lésion pulmonaire aiguë (ALI), du syndrome de détresse respiratoire aiguë (SDRA), de l'emphysème pulmonaire, du déficit en alpha 1-antitrypsine (DAAT), de la mucoviscidose, de la bronchiectasie, et permettant de favoriser la cicatrisation, notamment dans le cas de plaies chroniques.

7. Médicament contenant le composé répondant à la formule (I) sous la forme cristalline (A) selon l'une des revendications 1 et 2 à raison de plus de 90 pour cent en poids par rapport à la quantité totale du composé répondant à la formule (I) contenu.

8. Utilisation du composé répondant à la formule (I) sous la forme cristalline (A) selon l'une des revendications 1 et 2 pour produire un médicament permettant de traiter des troubles pulmonaires et du système cardiaque/circulatoire et permettant de favoriser la cicatrisation, notamment dans le cas de plaies chroniques.

9. Procédé selon la revendication 3 pour la production d'un composé répondant à la formule (I) sous la forme cristalline (A) selon l'une des revendications 1 et 2 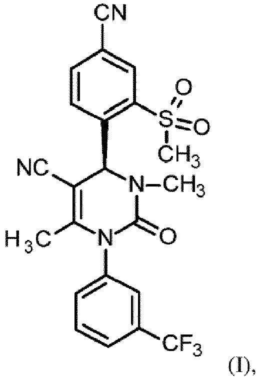 **caractérisé en ce que** l'on fait réagir un composé répondant à la formule (IX) 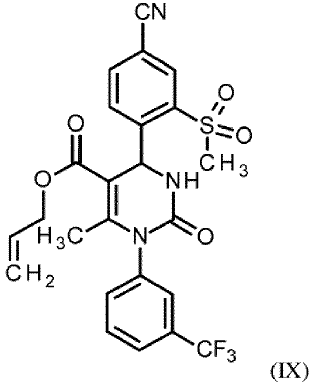
a-1) en présence d'un agent de méthylation et d'une base pour obtenir un composé répondant à la formule (XVI) 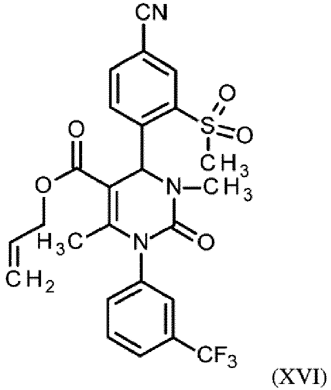 après quoi
a-2) on fait réagir un composé répondant à la formule (XVI) en présence d'un catalyseur palladium et d'une base amine secondaire pour obtenir un composé répondant à la formule (XXVI) 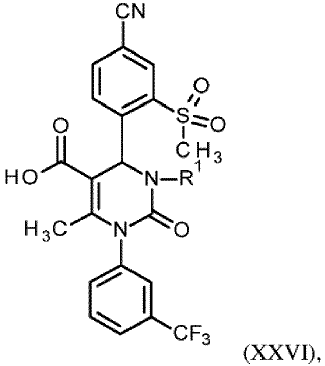 dans laquelle R¹ représente un méthyle,
ou
b-1) on le fait réagir en présence d'un catalyseur palladium et d'une base amine secondaire pour obtenir un composé répondant à la formule (XXVI) dans laquelle R¹ représente l'hydrogène ;
après quoi
c) on fait réagir un composé répondant à la formule (XXVI) dans laquelle R¹ représente l'hydrogène ou un méthyle, en présence d'un alcaloïde de quinquina et d'un solvant pour obtenir des composants répondant aux formules (XXVIII) et (XXIX) 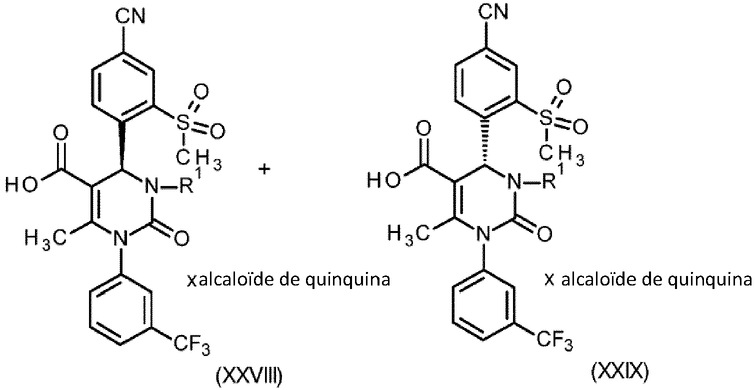 dans lesquelles R¹ dans la formule (XXVIII) et la formule (XXIX) représente l'hydrogène ou dans lesquelles R¹ dans la formule (XXVIII) et la formule (XXIX) représente un méthyle ; après quoi
d) on isole un composé répondant à la formule (XXVIII) ; après quoi
e) on fait réagir un composé répondant à la formule (XXVIII) en présence d'un acide fort pour obtenir un composé répondant à la formule (XXVII) 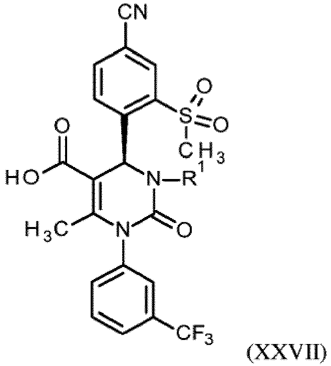 dans laquelle R¹ représente l'hydrogène ou un méthyle ; après quoi
b-2) lorsque, dans le composé répondant à la formule (XXVII), R¹ représente l'hydrogène, on fait réagir un composé répondant à la formule (XXVII) en présence d'un halogénure d'allyle ou sulfonate d'allyle et d'une base pour obtenir un composé répondant à la formule (X) 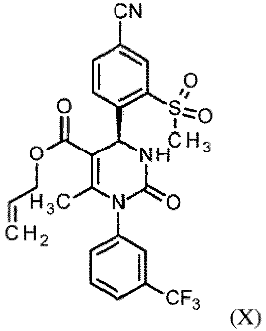 après quoi
b-3) on fait réagir un composé répondant à la formule (X) en présence d'un agent de méthylation et d'une base pour obtenir un composé répondant à la formule (XXIII) 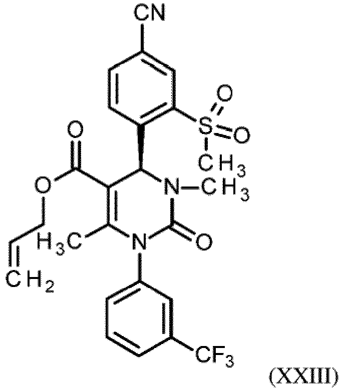 après quoi
b-4) on fait réagir un composé répondant à la formule (XXIII) en présence d'un catalyseur palladium et d'une base amine secondaire pour obtenir un composé répondant à la formule (XXVII) 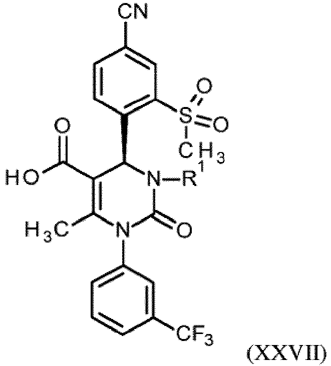 dans laquelle R¹ représente un méthyle ; après quoi
f) on fait réagir un composé répondant à la formule (XXVII) dans laquelle R¹ représente un méthyle, en présence d'un réactif d'activation pour obtenir un composé répondant à la formule (XIX) 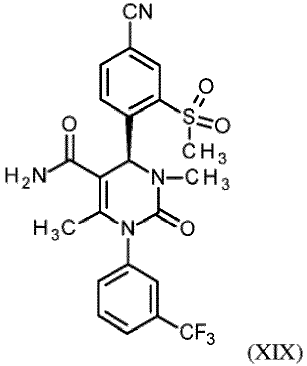 après quoi
g) on emploie un composé répondant à la formule (XIX) en présence d'un agent de déshydratation pour obtenir un composé répondant à la formule (I) ;
on isole le cas échéant, à la suite de l'étape réactionnelle c), un composé répondant à la formule (XXIX) 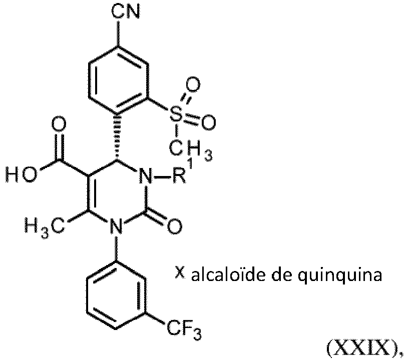 dans laquelle R¹ représente l'hydrogène, et on fait réagir ce dernier selon étape réactionnelle e) en présence d'un acide fort pour obtenir un composé répondant à la formule (XXX) 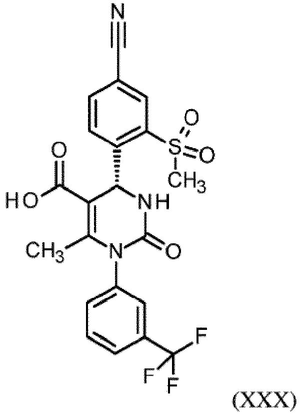 après quoi on fait réagir un composé répondant à la formule (XXX) selon l'étape réactionnelle b-2) en présence d'un halogénure d'allyle ou sulfonate d'allyle et d'une base pour obtenir un composé répondant à la formule (XXXI) 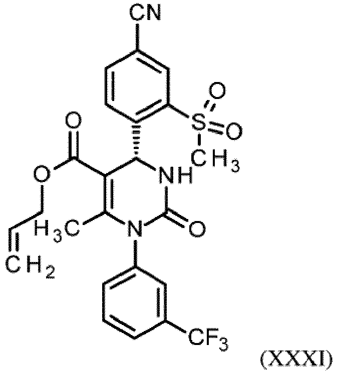 après quoi
h) on fait réagir un composé répondant à la formule (XXXI) en présence d'une base forte non nucléophile dans un solvant et sous chauffage simultané pour obtenir le racémate répondant à la formule (IX) 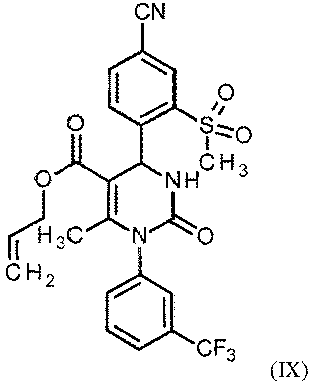 après quoi on fait réagir un composé répondant à la formule (IX) selon les étapes réactionnelles susdécrites b-1), c), d), e), b-2), b-3), b-4), f) et g) pour obtenir un composé répondant à la formule (I) ;
et le cas échéant on répète une ou plusieurs fois les étapes réactionnelles d'isolement d'un composé répondant à la formule (XXIX), sa réaction selon l'étape réactionnelle e) en présence d'un acide fort pour obtenir un composé répondant à la formule (XXX), la réaction subséquente d'un composé répondant à la formule (XXX) selon l'étape réactionnelle b-2) en présence d'un halogénure d'allyle ou sulfonate d'allyle et d'une base pour obtenir un composé répondant à la formule (XXXI), et la réalisation subséquente des étapes réactionnelles h), b-1), c), d), e), b-2), b-3), b-4), f) et g) ;
étant entendu que, le cas échéant, dans les formules (XXVI), (XXVII), (XXVIII) et (XXIX), R¹ représente un méthyle et que le procédé comprend les étapes réactionnelles a-1), a-2), c), d), e), f) et g) ;
étant entendu que, le cas échéant, dans les formules (XXVI), (XXVII), (XXVIII) et (XXIX), R¹ représente l'hydrogène et que le procédé comprend les étapes réactionnelles b-1), c), d), e), b-2), b-3), b-4), f) et g) ;
le cas échéant, **caractérisé en ce que** :
on isole, à la suite de l'étape réactionnelle c), un composé répondant à la formule (XXIX), on le met à réagir selon l'étape réactionnelle e) en présence d'un acide fort pour obtenir un composé répondant à la formule (XXX) ; après quoi on fait réagir un composé répondant à la formule (XXX) selon l'étape réactionnelle b-2) en présence d'un halogénure d'allyle ou sulfonate d'allyle et d'une base pour obtenir un composé répondant à la formule (XXXI) ; après quoi
h) on fait réagir un composé répondant à la formule (XXXI) en présence d'une base forte non nucléophile dans un solvant et sous chauffage simultané pour obtenir le racémate répondant à la formule (IX) ; après quoi
on fait réagir un composé répondant à la formule (IX) selon les étapes réactionnelles susdécrites b-1), c), d), e), b-2), b-3), b-4), f) et g) pour obtenir un composé répondant à la formule (I) ;
et, le cas échéant, on répète une ou plusieurs fois les étapes réactionnelles d'isolement d'un composé répondant à la formule (XXIX), sa réaction selon l'étape réactionnelle e) en présence d'un acide fort pour obtenir un composé répondant à la formule (XXX), la réaction subséquente d'un composé répondant à la formule (XXX) selon l'étape réactionnelle b-2) en présence d'un halogénure d'allyle ou sulfonate d'allyle et d'une base pour obtenir un composé répondant à la formule (XXXI), et la réalisation subséquente des étapes réactionnelles h), b-1), c), d), e), b-2), b-3), b-4), f) et g),
étant entendu que l'on cristallise le composé répondant à la formule (I) à partir de la phase organique issue de l'étape réactionnelle g) dans un alcool, de préférence l'éthanol, après quoi on chauffe la masse cristalline qui en résulte entre 50 et 80 °C et on agite pendant 2 à 5 h à cette température.
